# EUROPEAN PATENT APPLICATION

(11) **EP 4 047 010 A1**
(43) Date of publication of application: **24.08.2022**
(21) Application number: 20877197.2
(22) Date of filing: 16.10.2020
(51) Int. Cl.: C07K 7/08, C07K 16/00, C07K 19/00, A61K 51/10

(54) **METHOD FOR PRODUCING RADIOACTIVE METAL-LABELED ANTIBODY**

(30) Priority: 18.10.2019 JP 2019191561
(71) Applicant: Nihon Medi-Physics Co., Ltd., Tokyo 136-0075 (JP)
(72) Inventor: IZAWA, Akihiro, Tokyo 136-0075 (JP); OGAWA, Yu, Tokyo 136-0075 (JP); ICHIKAWA, Hiroaki, Tokyo 136-0075 (JP); KAWATANI, Minoru, Tokyo 136-0075 (JP); TAKEMORI, Hideaki, Tokyo 136-0075 (JP); OKUMURA, Yuki, Tokyo 136-0075 (JP)
(74) Representative: J A Kemp LLP
(86) International application number: PCT/JP2020/039076
(87) International publication number: WO 2021/075546

(57) **Abstract**

The production method of a radioactive metal-labeled antibody of the present invention includes a step of conducting a click reaction of a radioactive metal complex and an antibody site-specifically modified with a peptide to produce a radioactive metal-labeled antibody. The click reaction is performed between a first atomic group of the radioactive metal complex and a second atomic group directly or indirectly linked to the peptide. The second atomic group is an atomic group containing an azide group, or an atomic group containing trans-cyclooctene.

SEQUENCE LISTING

<110>  Nihon Medi-Physics Co., Ltd.

<120>  Method for the production of radioactive metal-labeled antibodies

<130>  093090

<150>  JP2019191561
<151>  2019-10-18

<160>  16

<170>  PatentIn version 3.5

<210>  1
<211>  13
<212>  PRT
<213>  Artificial Sequence

<220>
<223>  Peptide for modifying antibody cited in the specification,
       paragraph [0109] (1)


<220>
<221>  SITE
<222>  (6)..(6)
<223>  Xaa is a lysine residue, a cysteine residue, an aspartic acid
       residue, a glutamic acid residue, a 2-aminosuberic acid or
       diaminopropionic acid.

<400>  1

Asp Cys Ala Tyr His Xaa Gly Glu Leu Val Trp Cys Thr
1               5                   10


<210>  2
<211>  17
<212>  PRT
<213>  Artificial Sequence

<220>
<223>  Peptide for modifying antibody cited in the specification,
       paragraph [0109] (2)

<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  2

Gly Pro Asp Cys Ala Tyr His Xaa Gly Glu Leu Val Trp Cys Thr Phe
1               5                   10                  15


His

<210>  3
<211>  13
<212>  PRT
<213>  Artificial Sequence

<220>
<223>  Peptide for modifying antibody cited in the specification,
       paragraph [0109] (3)


<220>
<221>  SITE
<222>  (6)..(6)
<223>  Xaa is a lysine residue, a cysteine residue, an aspartic acid
       residue, a glutamic acid residue, a 2-aminosuberic acid or
       diaminopropionic acid.

<400>  3

Arg Cys Ala Tyr His Xaa Gly Glu Leu Val Trp Cys Ser
1               5                   10


<210>  4
<211>  17
<212>  PRT
<213>  Artificial Sequence

<220>
<223>  Peptide for modifying antibody cited in the specification,
       paragraph [0109] (4)


<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  4

Gly Pro Arg Cys Ala Tyr His Xaa Gly Glu Leu Val Trp Cys Ser Phe
1               5                   10                  15


His




<210>  5
<211>  17
<212>  PRT
<213>  Artificial Sequence

<220>
<223>  Peptide for modifying antibody cited in the specification,
       paragraph [0109] (5)


<220>
<221>  SITE

<222>  (8)..(8)
<223>  Xaa is a lysine residue, a cysteine residue, an aspartic acid
       residue, a glutamic acid residue, a 2-aminosuberic acid or
       diaminopropionic acid.

<400>  5

Ser Pro Asp Cys Ala Tyr His Xaa Gly Glu Leu Val Trp Cys Thr Phe
1               5                   10                  15

His

<210>  6
<211>  17
<212>  PRT
<213>  Artificial Sequence

<220>
<223>  Peptide for modifying antibody cited in the specification,
       paragraph [0109] (6)

<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  6

Gly Asp Asp Cys Ala Tyr His Xaa Gly Glu Leu Val Trp Cys Thr Phe
1               5                   10                  15

His

<210>  7
<211>  17
<212>  PRT
<213>  Artificial Sequence

<220>
<223>  Peptide for modifying antibody cited in the specification,
       paragraph [0109] (7)

<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  7

Gly Pro Ser Cys Ala Tyr His Xaa Gly Glu Leu Val Trp Cys Thr Phe
1               5                   10                  15

His




<210>  8
<211>  17
<212>  PRT
<213>  Artificial Sequence

<220>
<223>  Peptide for modifying antibody cited in the specification,
       paragraph [0109] (8)



<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  8

Gly Pro Asp Cys Ala Tyr His Xaa Gly Glu Leu Val Trp Cys Ser Phe
1               5                   10                  15


His




<210>  9
<211>  17
<212>  PRT
<213>  Artificial Sequence

<220>
<223>  Peptide for modifying antibody cited in the specification,
       paragraph [0109] (9)



<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  9

Gly Pro Asp Cys Ala Tyr His Xaa Gly Glu Leu Val Trp Cys Thr His
1               5                   10                  15


His




<210>  10
<211>  17
<212>  PRT

<213>  Artificial Sequence

<220>
<223>  Peptide for modifying antibody cited in the specification,
       paragraph [0109] (10)

<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  10

Gly Pro Asp Cys Ala Tyr His Xaa Gly Glu Leu Val Trp Cys Thr Phe
1               5                   10                  15

Tyr

<210>  11
<211>  17
<212>  PRT
<213>  Artificial Sequence

<220>
<223>  Peptide for modifying antibody cited in the specification,
       paragraph [0109] (11)

<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  11

Ser Pro Asp Cys Ala Tyr His Xaa Gly Glu Leu Val Trp Cys Thr Phe
1               5                   10                  15

Tyr

<210>  12
<211>  17
<212>  PRT
<213>  Artificial Sequence

<220>
<223>  Peptide for modifying antibody cited in the specification,
       paragraph [0109] (12)

<220>
<221>  SITE
<222>  (8)..(8)

<223>  Xaa is a lysine residue, a cysteine residue, an aspartic acid
       residue, a glutamic acid residue, a 2-aminosuberic acid or
       diaminopropionic acid.

<400>  12

Ser Asp Asp Cys Ala Tyr His Xaa Gly Glu Leu Val Trp Cys Thr Phe
1               5                   10                  15

Tyr

<210>  13
<211>  17
<212>  PRT
<213>  Artificial Sequence

<220>
<223>  Peptide for modifying antibody cited in the specification,
       paragraph [0109] (13)

<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<400>  13

Arg Gly Asn Cys Ala Tyr His Xaa Gly Gln Leu Val Trp Cys Thr Tyr
1               5                   10                  15

His

<210>  14
<211>  17
<212>  PRT
<213>  Artificial Sequence

<220>
<223>  Peptide for modifying antibody cited in the specification,
       paragraph [0109] (14)

<220>
<221>  SITE
<222>  (2)..(2)
<223>  Xaa is a homosysteine residue.

<220>
<221>  SITE
<222>  (8)..(8)
<223>  Xaa is a lysine residue, a cysteine residue, an aspartic acid
       residue, a glutamic acid residue, a 2-aminosuberic acid or
       diaminopropionic acid.

<220>
<221>  SITE
<222>  (16)..(16)
<223>  Xaa is a homosysteine residue.

<400>  14

Gly Xaa Asp Cys Ala Tyr His Xaa Gly Glu Leu Val Trp Cys Thr Xaa
1               5                   10                  15

His

<210>  15
<211>  17
<212>  PRT
<213>  Artificial Sequence

<220>
<223>  Peptide for modifying antibody cited in the specification, P3

<220>
<221>  MOD_RES
<222>  (1)..(1)
<223>  linking to linker structure

<220>
<221>  DISULFID
<222>  (4)..(14)

<220>
<221>  MOD_RES
<222>  (8)..(8)
<223>  modification with R1 structure

<400>  15

Gly Pro Asp Cys Ala Tyr His Lys Gly Glu Leu Val Trp Cys Thr Phe
1               5                   10                  15

His

<210>  16
<211>  17
<212>  PRT
<213>  Artificial Sequence

<220>
<223>  Peptide for modifying antibody cited in the specification, P4

<220>
<221>  MOD_RES
<222>  (1)..(1)
<223>  linking to linker structure

<220>
<221>  MOD_RES

<222>  (4)..(14)
<223>  linking via linker structure

<220>
<221>  MOD_RES
<222>  (8)..(8)
<223>  modification with R2 structure

<400>  16

Gly Pro Asp Cys Ala Tyr His Lys Gly Glu Leu Val Trp Cys Thr Phe
1               5                   10                  15

His

## Description

### TECHNICAL FIELD

The present invention relates to a production method of a radioactive metal-labeled antibody.

### BACKGROUND ART

An antibody is used as a reagent for detecting a target molecule, a diagnostic agent, or a pharmaceutical product for treating a disease by utilizing the specificity of the antibody for a target molecule. To further improve the detection performance and therapeutic effect, studies on antibodies bound to radionuclides and drugs are underway.

Patent Literature 1 describes a peptide containing an amino acid sequence consisting of 13 to 17 amino acid residues and capable of binding to a human antibody. This document also describes that the above-mentioned peptide can be modified with a labeling substance such as a complex containing a radioisotope or a drug such as an anticancer agent, and that a complex of the peptide and an antibody can be formed.

Patent Literature 2 describes a conjugate of an antibody and a peptide modified by the DTPA complex of ¹¹¹In, which is a radioactive metal. It is also described that this antibody conjugate specifically binds to a target molecule expressed in a tumor.

### CITATION LIST

### PATENT LITERATURES

Patent Literature 1: WO 2016/186206
Patent Literature 2: WO 2017/217347
Patent Literature 3: WO 2019/125982

### SUMMARY OF INVENTION

When producing an antibody bound with a radioactive metal complex, it may be necessary to heat the reaction system in order to increase the efficiency of complex formation, depending on the combination of the ligand and the radioactive metal to be used. However, under this condition, the desired antibody cannot be obtained since the antibody is denatured by heating. Patent Literatures 1 and 2 do not study the reaction conditions for solving such problem.

Therefore, the problem of the present invention is to provide a production method of a radioactive metal-labeled antibody superior in the labeling efficiency of radioactive metals for antibodies even under mild reaction conditions.

The present invention provides a method for producing a radioactive metal-labeled antibody comprising a step of conducting a click reaction of a radioactive metal complex and an antibody site-specifically modified with a peptide to produce the radioactive metal-labeled antibody, wherein the click reaction is performed between a first atomic group of the radioactive metal complex and a second atomic group directly or indirectly linked to the peptide, and the second atomic group is an atomic group containing an azide group, or an atomic group containing trans-cyclooctene.

In addition, the present invention provides a radioactive metal-labeled antibody site-specifically modified with a peptide,
wherein the radioactive metal complex is directly or indirectly linked to the peptide, and a triazole skeleton-containing structure represented by the following formula (10a) is present between the peptide and the radioactive metal complex, or a pyridazine skeleton-containing structure is present between the peptide and the radioactive metal complex: 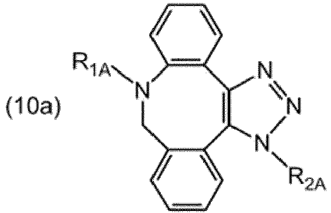 wherein R_{1A} is a binding site with a modified site or a chelate site, and R_{2A} is a binding site with a peptide.

In addition, the present invention provides a chelate linker comprising a chelate site coordinated to a radioactive metal, and a modified site having a first atomic group capable of click reaction, wherein the first atomic group is an atomic group represented by the following formula (1a) or (1b): 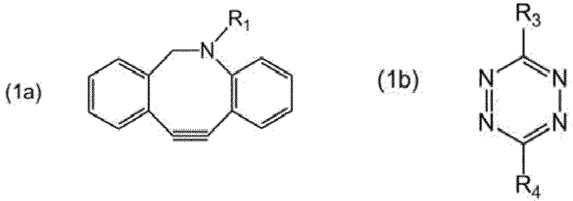 in the formula (1a), R₁ is a binding site with a modified site or a chelate site, and in the formula (1b), one of R₃ and R₄ is a binding site with a modified site or a chelate site, and the other is a hydrogen atom, a methyl group, a phenyl group or a pyridyl group.

In addition, the present invention provides a peptide-modified antibody represented by the following formula (i) and site-specifically modified by a peptide comprising an amino acid sequence consisting of 13 to 17 amino acid residues, which antibody comprising
a second atomic group capable of click reaction at the N-terminal or C-terminal of the peptide, wherein the second atomic group is an atomic group represented by the following formula (2a) or (2b):

(Xa)-Xaa1-(Xb)-Xaa2-(Xc)-Xaa3-(Xd)... (i)

wherein Xa, Xb, Xc, and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently an amino acid residue derived from an amino acid having a thiol group in the side chain, and they are bonded via a disulfide bond or their sulfide groups are bonded via a linker, or
one is an amino acid residue derived from an amino acid having a thiol group in the side chain and the other is an amino acid residue derived from an amino acid having a haloacetyl group in the side chain, and they are bonded via a thioether bond, and Xaa2 is a lysine residue, an arginine residue, a cysteine residue, an aspartic acid residue, a glutamic acid residue, a 2-aminosuberic acid, or a diamino propionic acid,

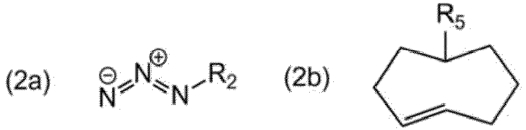

in the formula (2a), R₂ is a binding site with the N-terminal or C-terminal of the peptide, and in the formula (2b), R₅ is a binding site with the N-terminal or C-terminal of the peptide.

According to the present invention, the labeling efficiency of a radioactive metal for an antibody is superior even under mild reaction conditions.

### DESCRIPTION OF EMBODIMENTS

In the following, the production method of a radioactive metal-labeled antibody of the present invention is explained based on preferred embodiments thereof.

The production method of the present invention includes a step of producing a radioactive metal-labeled antibody (labeling step) by a click reaction of a radioactive metal complex and an antibody site-specifically modified with a peptide (hereinafter to be also simply referred to as a "peptide-modified antibody"). The details of the radioactive metal and a complex thereof, and the peptide are described later.

The radioactive metal complex and the peptide-modified antibody each have an atomic group capable of click reaction, and these atomic groups react with each other to allow the radioactive metal complex and the peptide-modified antibody to bind to each other. That is, the click reaction in this step is performed between the first atomic group contained in the radioactive metal complex and the second atomic group directly or indirectly linked to the peptide in the peptide-modified antibody.

The "directly or indirectly" refers to whether there is a linker structure described later between the second atomic group and the peptide. "Directly" means not having a linker structure, and more specifically means that the second atomic group is bonded to the N-terminal or C-terminal of the peptide not via a linker structure. "Indirectly" means having a linker structure, and more specifically means that the second atomic group is bonded to the N-terminal or C-terminal of the peptide via a linker structure. In either "directly" or "indirectly" embodiment, the second atomic group is preferably bonded to the N-terminal side of the peptide.

The aforementioned linker structure is represented by the following formula (S1).

**-((L₁)ₘ-Z)ₖ-L₂-AG₂... (S1)

wherein ** is a binding site with the N-terminal or C-terminal of a peptide,
L₁ is a linker site of polyethylene glycol (PEG),
m is an integer of not less than 1 and not more than 50,
Z is a second linker site that binds (L₁)ₘ and L₂,
k is 0 or 1,
L₂ is a second PEG linker site, and
AG₂ is a second atomic group.

In the aforementioned formula (S1), the structure of Z is not particularly limited as long as it is a linker structure that binds (L₁)ₘ and L₂ to each other, and includes, for example, an amino acid sequence consisting of not less than 1 and not more than 5 amino acid residues. In this case, the amino acid sequence contained in Z preferably contains a cysteine residue, and is more preferably bonded to L₂ via a thioether group formed by the bond between the thiol group of the cysteine residue and a maleimide group.

In the present invention, the PEG linker site constituting L₂ preferably has the structure shown by the following formula (P1). In the formula (P1), n is an integer of preferably not less than 1 and not more than 50, more preferably not less than 1 and not more than 20, further preferably not less than 2 and not more than 10.

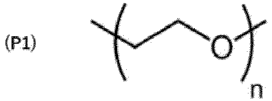

One end of the structure of the PEG linker site may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

In the present step, as the combination of the atomic groups capable of click reaction, an appropriate combination is selected according to the type of the click reaction. For example, a combination of alkyne and azide, a combination of 1,2,4,5-tetrazine and alkene, and the like can be mentioned. In these atomic groups, it is sufficient if the first atomic group has one of the above-mentioned atomic groups and the second atomic group has an atomic group to be a combination with the first atomic group. To achieve both the stability of radioactive metal complex and peptide-modified antibody and the improvement of the binding efficiency thereof, the first atomic group is preferably alkyne and the second atomic group is preferably azide, or the first atomic group is preferably 1,2,4,5-tetrazine and the second atomic group is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), or else a combination of an atomic group containing 1,2,4,5-tetrazine as the first atomic group (the formula (1b)) and an atomic group containing trans-cyclooctene (TCO) as alkene of the second atomic group (the formula (2b)).

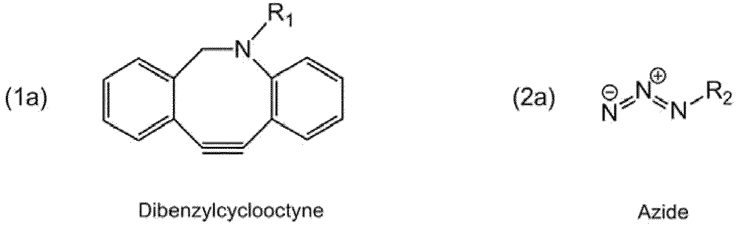

(in the formula (1a), R₁ is a binding site with a modified site or a chelate site, and in the formula (2a), R₂ is a peptide binding site of the peptide-modified antibody.)

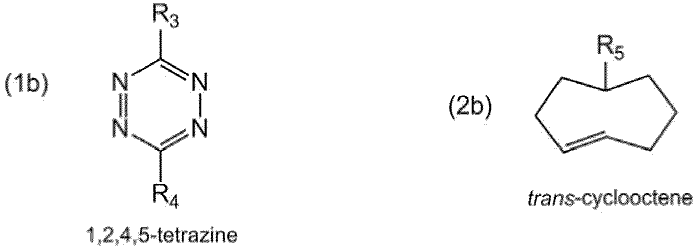

(in the formula (1b), one of R₃ and R₄ is a binding site with other structure, and the other is a hydrogen atom, a methyl group, a phenyl group or a pyridyl group, and in the formula (2b), R₅ is a binding site with other structure.)

In this step, when the radioactive metal complex and the peptide-modified antibody are capable of click reaction, the order of addition of these does not matter. For example, one of the radioactive metal complex and the peptide-modified antibody is added to a reaction container containing a solvent, and then the other is added to perform the reaction, or one of the radioactive metal complex and the peptide-modified antibody is dispersed in a solvent and the other is added to the dispersion to perform the reaction. Alternatively, these may be simultaneously added to a reaction container containing a solvent to perform the reaction.

As the solvent to be used in this step, solvents containing water can be used. For example, water, saline, or buffer such as sodium acetate buffer, ammonium acetate buffer, phosphate buffer, phosphate buffered saline, tris hydroxymethylaminomethane buffer (hereinafter to be simply referred to as "Tris buffer"), 4-(2-hydroxyethyl)-1-piperazineethanesulfonic acid buffer (hereinafter to be simply referred to as "HEPES buffer"), tetramethylammonium acetate buffer or the like can be used. When a buffer is used, in order to simultaneously achieve both the stability of complex and antibody, and the binding efficiency thereof, the lower limit of pH at 25°C is preferably not less than 3.5, more preferably not less than 4.0, further preferably not less than 4.5, further more preferably not less than 5.0, particularly preferably not less than 5.5, the upper limit is preferably not more than 10.0, more preferably not more than 9.5, further preferably not more than 9.0, further more preferably not more than 8.5, particularly preferably not more than 8.0, and a preferable range is not less than 4.0 and not more than 10.0, more preferably not less than 5.5 and not more than 8.5.

In order to enhance the binding efficiency between a radioactive metal complex and a peptide-modified antibody while avoiding unintended denaturation of the peptide-modified antibody, the upper limit of the reaction temperature of the click reaction in this step is preferably not more than 120°C, more preferably not more than 90°C, further preferably not more than 50°C, further more preferably not more than 40°C. The lower limit of the reaction temperature is not particularly limited as long as it is a temperature at which the click reaction can be performed. It is preferably not less than 10°C, more preferably not less than 15°C, further preferably not less than 20°C, further more preferably not less than 30°C, particularly preferably not less than 35°C. On the condition that the aforementioned reaction temperature applies, the lower limit of the reaction time of the click reaction is preferably not less than 5 min, more preferably not less than 10 min, further preferably not less than 20 min, further more preferably not less than 30 min, particularly preferably not less than 60 min, and the upper limit is preferably not more than 36 hr, more preferably not more than 24 hr, further more preferably not more than 20 hr, particularly preferably not more than 15 hr, and a preferable range is not less than 5 min and not more than 24 hr, more preferably not less than 10 min and not more than 20 hr.

The amount of the reaction solution is not particularly limited. From the aspect of practicality in the production step, the lower limit is preferably not less than 0.01 mL, more preferably not less than 0.1 mL, further preferably not less than 1 mL, and the upper limit is preferably not more than 1000 mL, more preferably not more than 100 mL, further preferably not more than 10 mL, both at the start of this step. For example, it is preferably not less than 0.1 mL and not more than 10 mL. In addition, the concentrations of the radioactive metal complex and the peptide-modified antibody in the reaction solution are each independently preferably not less than 0.01 µmol/L, more preferably not less than 0.1 µmol/L, further preferably not less than 1 µmol/L as the lower limit, and preferably not more than 10000 µmol/L, more preferably not more than 1000 µmol/L, further preferably not more than 100 µmol/L, as the upper limit, both at the start of this step. For example, it is preferably not less than 1 µmol/L and not more than 100 µmol/L, from the aspect of the yield of the desired radioactive metal-labeled antibody.

The obtained radioactive metal-labeled antibody may be used as it is, or may be purified by using a filtration filter, a membrane filter, a column packed with various fillers, chromatography or the like.

According to the production method of the present invention including the aforementioned steps, a radioactive metal complex and a peptide-modified antibody are bonded under mild reaction conditions to obtain a radioactive metal-labeled antibody in a high yield. Since the obtained labeled antibody can be specifically labeled at a site that does not inhibit the antigen specificity of the antibody, the antigen specificity of the antibody itself is maintained. In particular, in the above-mentioned step, the binding reaction between the radioactive metal complex and the peptide-modified antibody can proceed sufficiently and in a short time without performing a heat treatment for promoting the binding reaction between the complex and the antibody. Therefore, even when a radioactive metal having a short half-life such as a positron-emitting nuclide is used, a labeled antibody having high radiochemical purity and high radiochemical yield can be obtained in a short time.

The production method of the present invention preferably includes a step (complex formation step) of reacting the ligand with the radioactive metal to form a radioactive metal complex before the labeling step, so that the radioactive metal used for labeling can be used according to the use and object of the labeled antibody without particular limitation. The ligand used in this step preferably has a first atomic group. In addition, from the aspect of increasing the complex formation efficiency, the radioactive metal in this step is preferably used in the form of an ionizable radioactive metal compound, further preferably in the form of a radioactive metal ion (hereinafter, these embodiments are collectively referred to as a "radioactive metal source").

From the aspect of increasing the formation efficiency of the radioactive metal complex without depending on the combination of the ligand and the radioactive metal, it is preferable to heat and react the ligand and the radioactive metal in the complex formation step. The complex formation may not proceed well under non-heating conditions, depending on the combination of the ligand and the radioactive metal. However, by forming under heating conditions a complex with a radioactive metal ion coordinated therein, the complex formation can be efficiently performed without depending on the combination of the ligand and the radioactive metal.

In the complex formation step, when a complex with a radioactive metal ion can be formed, the order of addition of the ligand and the radioactive metal source does not matter. For example, one of the ligand and the radioactive metal source is added to a reaction container containing a solvent, and then the other is added to perform the reaction, or one of the ligand and the radioactive metal source is dispersed in a solvent and the other is added to the dispersion to perform the reaction. Alternatively, these may be simultaneously added to a reaction container containing a solvent to perform the reaction.

As the reaction conditions in the complex formation step, for example, the following conditions can be adopted. As the solvent to be used in this step, water, saline, or a buffer such as sodium acetate buffer, ammonium acetate buffer, phosphate buffer, phosphate buffered saline, Tris buffer, HEPES buffer, or tetramethylammonium acetate buffer, or a watersoluble organic solvent such as alcohol with a carbon number of not less than one and not more than 5, acetonitrile, N,N-dimethylformamide, tetrahydrofuran, dimethyl sulfoxide, acetone or the like, or a mixed solvent thereof can be used. The reaction temperature may be, for example, room temperature (25°C) or under heating conditions. In order to simultaneously suppress decomposition of the ligand and improve the formation efficiency of the complex, the upper limit is preferably not more than 120°C, more preferably not more than 90°C, further preferably not more than 50°C, more preferably not more than 40°C. The lower limit is not particularly limited as long as it is a temperature at which the click reaction can be performed. It is preferably not less than 0°C, more preferably not less than 10°C, further preferably not less than 15°C, further more preferably not less than 20°C, still more further preferably not less than 30°C, particularly preferably not less than 35°C. Heating is performed at preferably not less than 30°C and not more than 100°C, further preferably not less than 37°C and not more than 90°C. On the condition that the aforementioned reaction temperature applies, the lower limit of the reaction time is preferably not less than 5 min, more preferably not less than 10 min, further preferably not less than 20 min, further more preferably not less than 30 min, particularly preferably not less than 60 min, and the upper limit is preferably not more than 300 min, more preferably not more than 150 min, further preferably not more than 120 min, particularly preferably not more than 60 min. It is preferably not less than 10 min and not more than 150 min, further preferably not less than 30 min and not more than 60 min.

As the radioactive metal source in the complex formation step, for example, a solution of a radioactive metal ion dispersed or dissolved in a solvent mainly containing water can be used.

The amount of the reaction solution is not particularly limited. From the aspect of practicality in the production step, the lower limit is preferably not less than 0.01 mL, more preferably not less than 0.1 mL, further preferably not less than 1 mL, and the upper limit is preferably not more than 1000 mL, more preferably not more than 100 mL, further preferably not more than 10 mL, both at the start of this step. For example, it is preferably not less than 0.01 mL and not more than 100 mL. In addition, the concentrations of the ligand and the radioactive metal ion in the reaction solution are each independently preferably not less than 0.01 µmol/L, more preferably not less than 0.1 µmol/L, further preferably not less than 1 µmol/L as the lower limit, and preferably not more than 10000 µmol/L, more preferably not more than 1000 µmol/L, further preferably not more than 100 µmol/L, as the upper limit, both at the start of this step. For example, it is preferably not less than 1 µmol/L and not more than 100 µmol/L, from the aspect of the yield of the desired radioactive metal complex. The molar ratios of the ligand and the radioactive metal ion vary depending on the kind of the ligand and radioactive metal ion to be used. As the ligand/radioactive metal ion, the lower limit is preferably not less than 10/1, more preferably not less than 100/1, not less than 200/1, further preferably not less than 300/1, further more preferably not less than 500/1, and the upper limit is preferably not more than 10000/1, more preferably not more than 9000/1, further preferably not more than 8000/1, further more preferably not more than 7000/1. It is preferably within the range of not less than 200/1 and not more than 10000/1, particularly preferably not less than 500/1 and not more than 7000/1.

The obtained radioactive metal complex may be used as it is, or may be purified by using a filtration filter, a membrane filter, a column packed with various fillers, chromatography or the like. In particular, in this step, even when low-energy radiation which is difficult to detect or a radioactive metal nuclide that emits α-ray is used, complex formation proceeds well and the yield of the resultant product is high. Thus, a complex containing the radioactive metal nuclide can be advantageously subjected to the subsequent steps in an unpurified state.

From the aspects of improving the reaction efficiency of the reaction between the ligand and the radioactive metal ion, and improving the reaction efficiency of the click reaction between the obtained complex and the peptide-modified antibody, it is preferable that the ligand has a chelate site where a radioactive metal ion is coordinated and a modified site bonded to the first atomic group.

As shown by the following formula (3a), the modified site (Rm in the formula (3a)) is bonded to the aforementioned chelate site (Ch in the formula (3a)), and also bonded to the aforementioned first atomic group (AG in the formula (3a)). In the formula (3a), Rm is a straight chain or branched chain, substituted or unsubstituted atomic group having a total carbon atom number of not less than 10 and not more than 50.

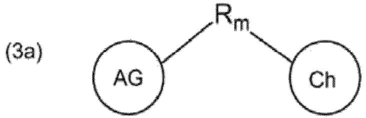

The modified site Rm is not particularly limited in the binding mode with the chelate site Ch as long as the complex can be formed between the ligand and the radioactive metal ion. From the aspect of efficiently forming a complex between the ligand and the radioactive metal ion, it is preferable that the modified site Rm and the chelate site Ch form a thiourea bond and bind to each other, or the modified site Rm and the chelate site Ch form an amide bond and bind to each other. From the aspect of increasing the labeling efficiency of the radioactive metal complex and the antibody, the modified site Rm is also preferably bound to R₁, and R₃ or R₄ represented by the above-mentioned formulas (1a) and (1b) in the first atomic group.

From the aspect of further increasing the labeling efficiency of the radioactive metal complex and the antibody, it is preferable that a structure represented by the following formula (P2) is bonded to the modified site of a structure represented by Rm in the formula (3a). The structure of the formula (P2) is derived from ethylene glycol, and in the formula (P2), r is preferably an integer of not less than 2 and not more than 50, further preferably not less than 2 and not more than 30.

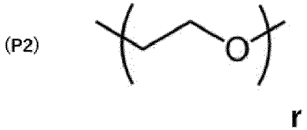

The chelate site is not particularly limited as long as it has, in the structure thereof, a site where radioactive metal is coordinated. It is preferably represented by CB-TE2A(1,4,8,11-Tetraazabicyclo[6.6.2]hexadecane-4,11-diacetic acid), CDTA(Cyclohexane-trans-1,2-diamine tetra-acetic acid), CDTPA(4-cyano-4-[[(dodecylthio)thioxomethyl]thio]-Pentanoic acid), DOTA(1,4,7,10-Tetraazacyclododecane-1,4,7,10-tetraacetic acid), DOTMA((1R,4R,7R,10R)-α,α′,αʺ,α‴-tetramethyl-1,4,7,10-tetraazacyclododecane-1,4,7,10-tetraacetic acid), DOTAM(1,4,7,10-tetrakis(carbamoylmethyl)-1,4,7,10-tetraazacyclododecane), DOTA-GA(α-(2-Carboxyethyl)-1,4,7,10-tetraazacyclododecane-1,4,7,10-tetraacetic acid), DOTP(((1,4,7,10-Tetraazacyclododecane-1,4,7,10-tetrayl)tetrakis(methylene))tetraphosphonic acid), DOTMP(1,4,7,10-Tetraazacyclododecane-1,4,7,10-tetrakis(methylenephosphonic acid)), DOTA-4AMP(1,4,7,10-tetraazacyclododecane-1,4,7,10-tetrakis(acetamidomethylenephosphonic acid), D02P(Tetraazacyclododecane dimethanephosphonic acid), Deferoxamine (DFO), DTPA(Glycine, N,N-bis[2-[bis(carboxymethyl)amino]ethyl]-), DTPA-BMA(5,8-Bis(carboxymethyl)-11-[2-(methylamino)-2-oxoethyl]-3-oxo-2,5,8,11-tetraazatridecan-13-oic acid), EDTA(2,2′,2ʺ,2‴-(ethane-1,2-diylbis(azanetriyl))tetraacetic acid), NOTA(1,4,7-Triazacyclononane-1,4,7-triacetic acid), NOTP(1,4,7-Triazacyclononane-1,4,7-triyltris(methylenephosphonic acid), TETPA(1,4,8,11-tetraazacyclotetradecane-1,4,8,11-tetrapropionic acid), TETA(1,4,8,11-Tetraazacyclotetradecane-N,N′,Nʺ,N‴-tetraacetic acid), TTHA(3,6,9,12-Tetrakis(carboxymethyl)-3,6,9,12-tetraazatetradecanedioic acid), HEHA(1,2,7,10,13-hexaazacyclooctadecane-1,4,7,10,13,16-hexaacetic acid), 1,2-HOPO(N,N′,Nʺ,N‴-tetra(1,2-dihydro-1-hydroxy-2-oxopyridine-6-carbonyl)-1,5,10,14-tetraazatetradecane), PEPA(1,4,7,10,13-pentaazacyclopentadecane-N,N′,Nʺ,N‴,Nʺʺ-penta-acetic acid), H4octapa(N,N'-bis(6-carboxy-2-pyridylmethyl)-ethylenediamine-N,N'-diacetic acid), H2bispa2(6,6'-({9-hydroxy-1,5-bis(methoxycarbonyl)-2,4-di(pyridin-2-yl)-3,7-diazabicyclo[3.3.1]nonane-3,7-diyl}bis(-methylene))dipicolinic acid), H2dedpa(1,2-[{6-(carboxy)-pyridin-2-yl}-methylamino]ethane), H2macropa(6-(1,4,10,13-tetraoxa-7,16-diazacyclooctadecan-N,N'-methyl)picolinic acid), H5decapa(N,Nʺ-bis(6-carboxy-2-pyridylmethyl)-diethylenetriamine-N,N′,Nʺ-triacetic acid), H6phospa(N,N'-(methylenephosphonate)-N,N'-[6-(methoxycarbonyl)pyridin-2-yl]-methyl-1,2-diaminoethane), HP-D03A(Hydroxypropyltetraazacyclododecanetriacetic acid), or porphyrin, or any one of the following formulas (A) to (K). These structures can be appropriately selected according to the kind of the radioactive metal described later. The effect of the present invention is sufficiently achieved using any chelate site.

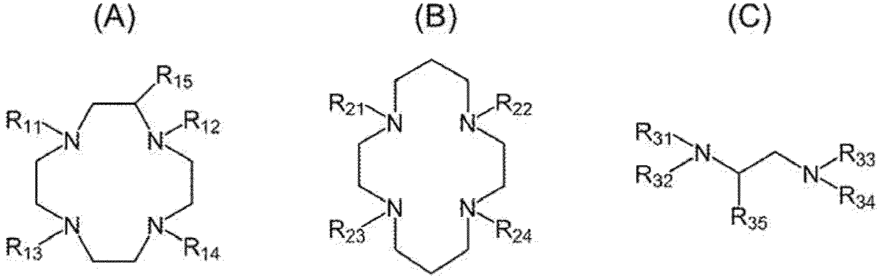

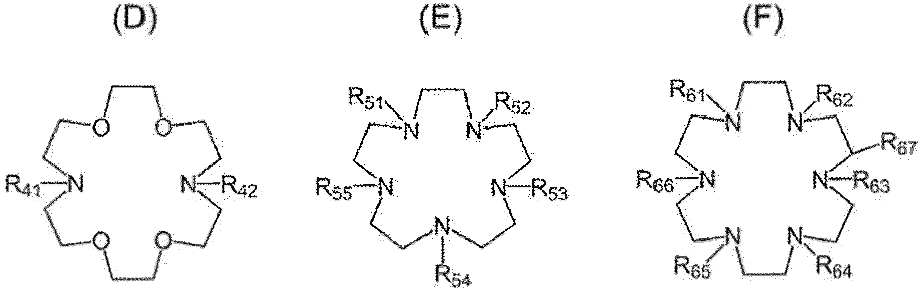

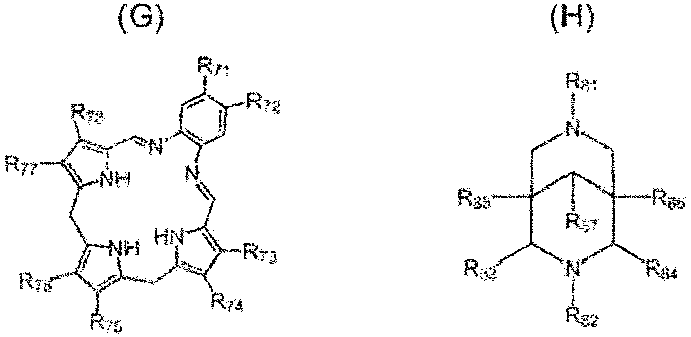

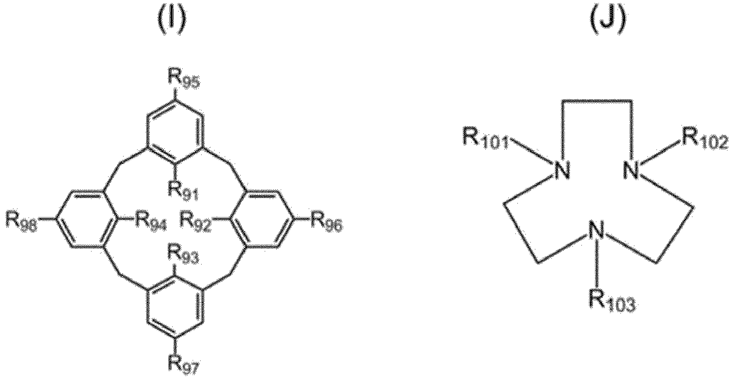

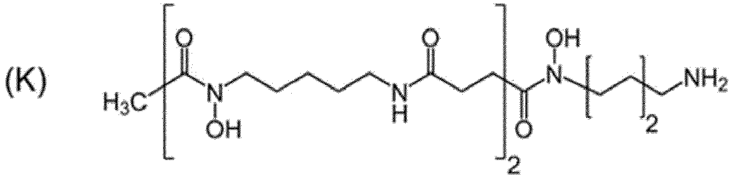

In the formula (A), R₁₁, R₁₃ and R₁₄ are each independently a group consisting of -(CH₂)ₚCOOH, -(CH₂)ₚC₅H₅N, -(CH₂)ₚPO₃H₂, - (CH₂)ₚCONH₂ or -(CHCOOH)(CH₂)ₚCOOH, one of R₁₂ and R₁₅ is a hydrogen atom, a carboxyl group, or a carboxyalkyl group having 2 or 3 carbon atoms, the other is a group bonded to the above-mentioned modified site, and p is an integer of not less than 0 and not more than 3.

In the formula (B), R₂₁, R₂₂, R₂₃ and R₂₄ are each independently a carboxyl group or a carboxyalkyl group having a carbon number of 2 or 3, and any one group of R₂₁, R₂₂, R₂₃ or R₂₄ is bonded to the above-mentioned modified site.

In the formula (C), R₃₁, R₃₂, R₃₃ and R₃₄ are each independently a group composed of an atomic group having a hydrogen atom and not less than 2 and not more than 10 carbon atoms, and optionally containing a nitrogen atom or an oxygen atom, and R₃₅ is a group bonded to the above-mentioned modified site.

In the formula (D), one of R₄₁ and R₄₂ is a group composed of an atomic group having a hydrogen atom and not less than 5 and not more than 20 carbon atoms and containing one or more kinds selected from nitrogen atom, oxygen atom and sulfur atom, and the other is a group bonded to the above-mentioned modified site.

In the formula (E), R₅₁, R₅₂, R₅₃, R₅₄ and R₅₅ are each independently a carboxyl group, or a carboxyalkyl group having a carbon atom number of 2 or 3, and any one group of R₅₁, R₅₂, R₅₃, R₅₄ or R₅₅ is bonded to the above-mentioned modified site.

In the formula (F), R₆₁, R₆₂, R₆₃, R₆₄, R₆₅ and R₆₆ are each independently a carboxyl group, or carboxyalkyl group having a carbon atom number of 2 or 3, and R₆₇ is a group bonded to the above-mentioned modified site.

In the formula (G), R₇₁ and R₇₂ are -O(CH₂CH₂O)ₙCH₃ (n is an integer of not less than 1 and not more than 5), R₇₃, R₇₅, R₇₆ and R₇₈ are each independently an alkyl group having a carbon atom number of not less than 1 and not more than 5, one of R₇₄ and R₇₇ is a hydroxyalkyl group having a carbon number of not less than 1 and not more than 5, and the other is a group bonded to the above-mentioned modified site.

In the formula (H), R₈₁ and R₈₂ are each independently an alkyl group having a carbon number of not less than 1 and not more than 5, a terminal of the alkyl group may be substituted by a pyridyl group substituted by one or more carboxyl groups, R₈₇ is -CHOH or -C=O, any one group of R₈₁, R₈₂ or R₈₇ is a group bonded to the above-mentioned modified site, R₈₃ and R₈₄ are optionally substituted pyridinyl groups, R₈₅ and R₈₆ are each independently -COORa, and Ra is an alkyl group having a carbon number of not less than 1 and not more than 5.

In the formula (I), R₉₁, R₉₂, R₉₃ and R₉₄ are each independently -OCH₂COOH, any one group of R₉₁, R₉₂, R₉₃ or R₉₄ is a group bonded to the above-mentioned modified site, and R₉₅, R₉₆, R₉₇ and R₉₈ are each independently an alkyl group having a carbon number of not less than 1 and not more than 6.

In the formula (J), R₁₀₁, R₁₀₂ and R₁₀₃ are each independently a carboxyl group or a carboxyalkyl group having a carbon atom number of 2 or 3, or at least one of R₁₀₁, R₁₀₂ and R₁₀₃ in the formula (J) is a group bonded to the above-mentioned modified site and the other group is a carboxyl group, or a carboxyalkyl group having a carbon atom number of 2 or 3.

In the above-mentioned formulas (A) to (J), the "group bonded to the modified site" is a structure derived from a carboxyl group, an amino group, an N-hydroxysuccinimidoester (NHS) group, a 2,6-dioxotetrahydro-2H-pyranyl group, an isocyanate group, or a structure of an isothiocyanate group bonded to a modified site.

Examples of the specific structure represented by the formula (A) include structures derived from the compounds represented by the following formulas (A-1) to (A-12).

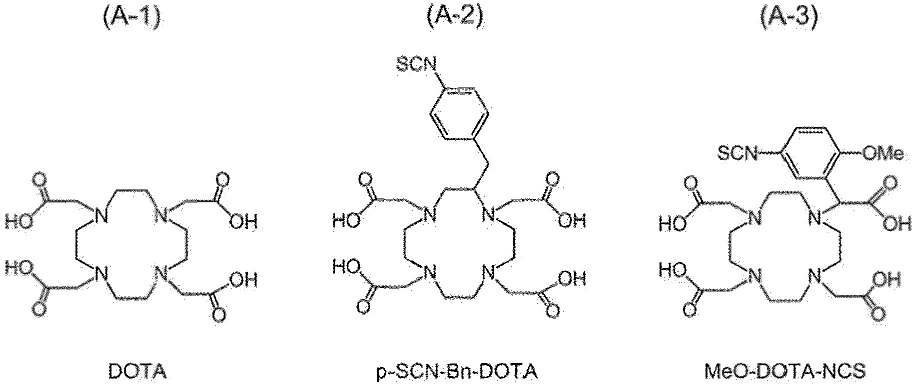

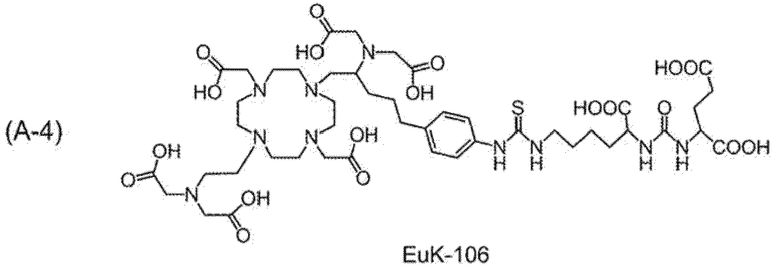

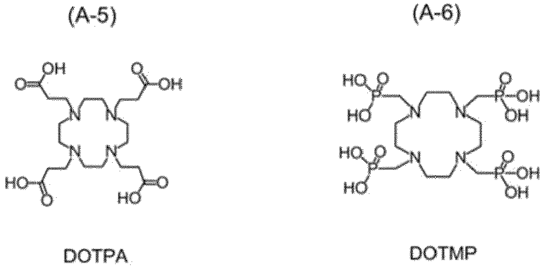

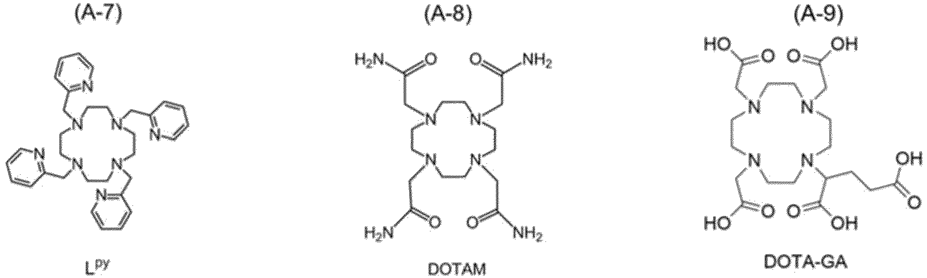

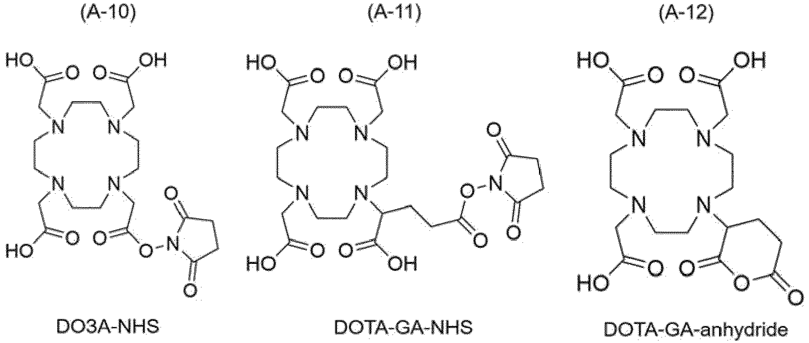

Examples of the specific structure represented by the formula (B) or (C) include structures derived from a compound represented by the following formulas (B-1) to (B-2) or (C-1) to (C-5).

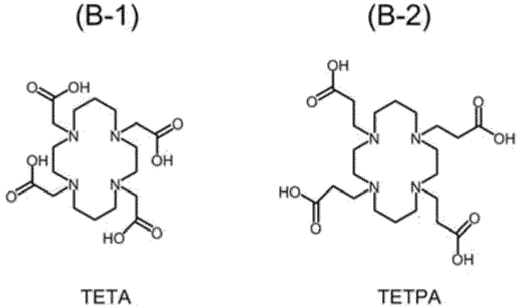

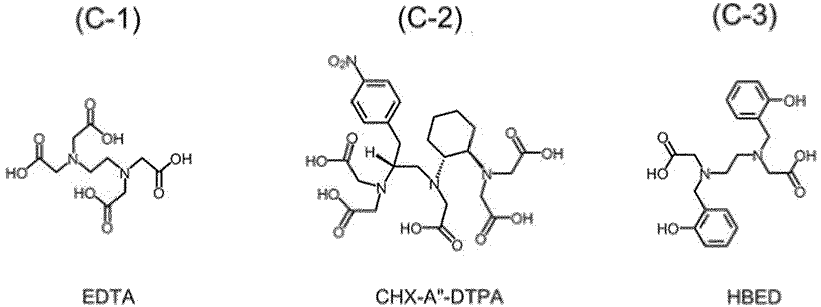

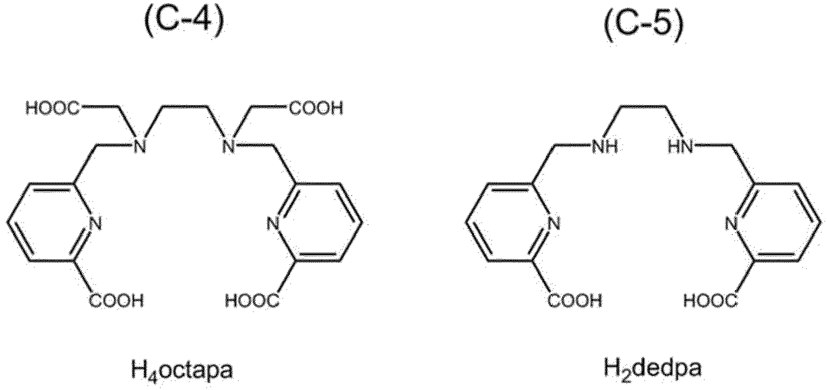

Examples of the specific structure represented by the formula (D) or (E) include structures derived from a compound represented by the following formulas (D-1) to (D-3), or (E-1).

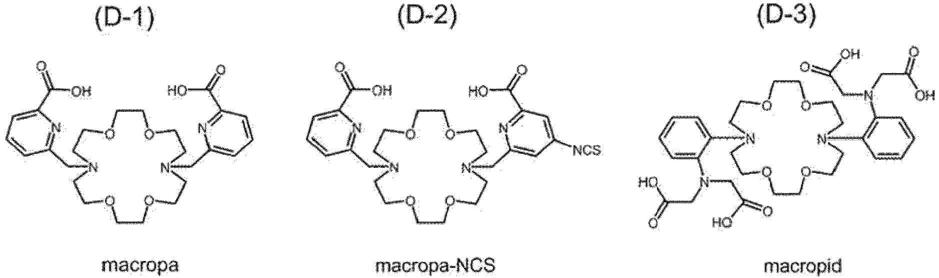

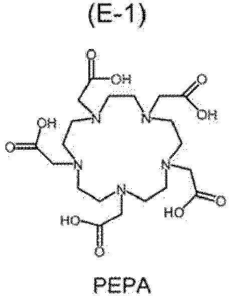

Examples of the specific structure represented by the formula (F) or (G) include structures derived from a compound represented by the following formulas (F-1) to (F-2) or (G-1).

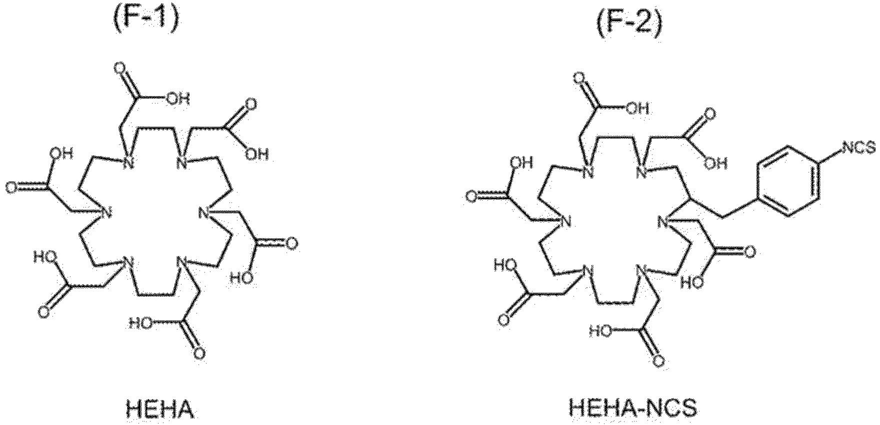

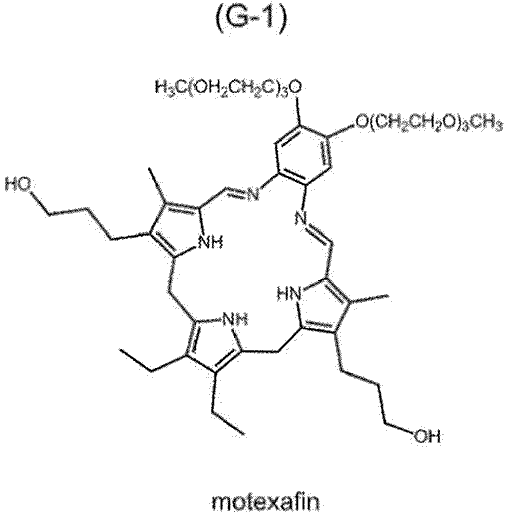

Examples of the specific structure represented by the formula (H) or (I) include structures derived from a compound represented by the following formulas (H-1) to (H-4), or (I-1).

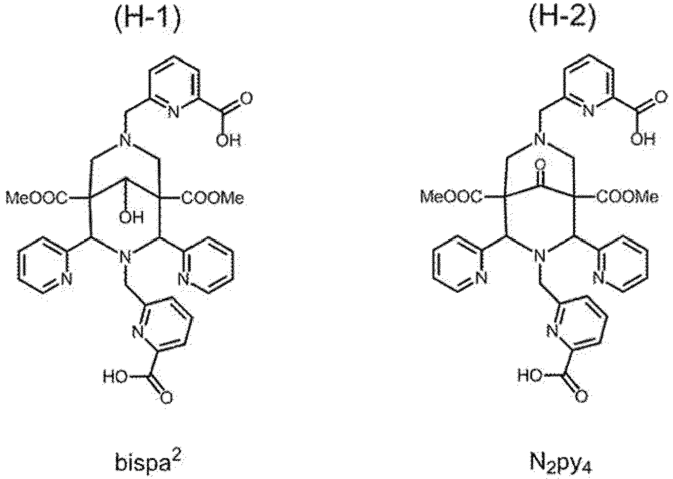

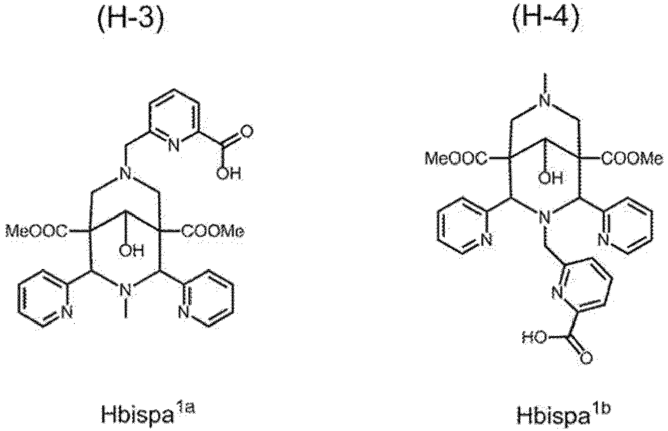

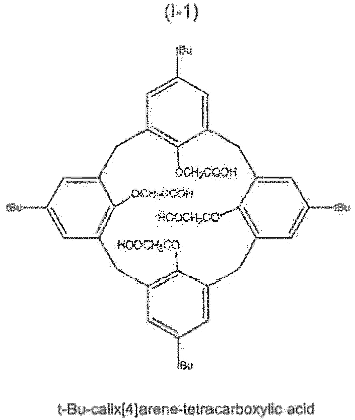

Examples of the specific structure represented by the formula (J) include structures derived from a compound represented by the following formulas (J-1) to (J-5).

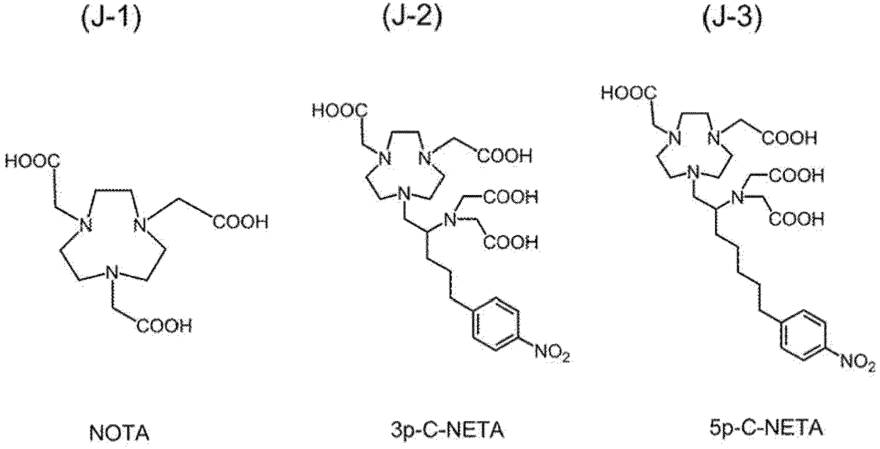

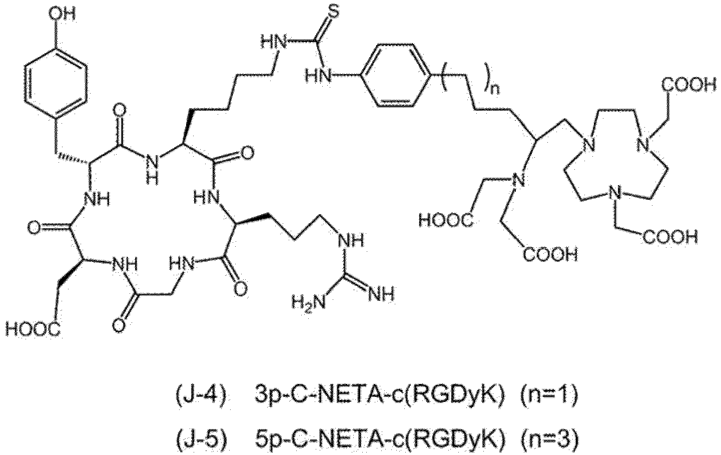

The binding site between the chelate site and the modified site is preferably an amide bond or a thiourea bond as mentioned above, more preferably an amide bond from the aspect of yield.

The amide bond can be formed, for example, by the reaction of a carboxyl group of the compound of the above-mentioned formula (B-1) to (B-2), (G-1), (H-1) to (H-4), (I-1), (J-1) to (J-3), an N-hydroxysuccinimidoester (NHS) group of the above-mentioned formula (A-10) or (A-11), or a 2,6-dioxo tetrahydro-2H-pyranyl group of the above-mentioned (A-12), with primary amine. Alternatively, the amide bond can be formed, for example, by the reaction of an amino group at the right end of the compound represented by the formula (K), and a reagent having a hydroxy group, a carboxyl group, or an NHS group. When a reagent having a hydroxy group is reacted, the hydroxy group is converted to a carboxyl group before use. The thiourea bond can be formed by the reaction of an isothiocyanate group of the compound of the above-mentioned formula (A-2), (A-3), (D-2), or (F-2), with primary amine or a maleimide group.

The modified site can be formed by variously selecting from commercially available reagents containing a primary amine or commercially available reagents capable of forming an amide bond or a thiourea bond, to all of which a desired first atomic group is bonded.

As such a reagent, when Dibenzylclocityne (DBCO) represented by the above-mentioned formula (1a) is used as the first atomic group, DBCO-amine, DBCO-maleimide, DBCO-PEG-NHS ester, DBCO-PEG-alcohol, DBCO-PEG-amine, DBCO-PEG-maleimide and the like, preferably DBCO-amine, DBCO-maleimide, DBCO-PEG-amine, DBCO-PEG-maleimide, can be selected.

The radioactive metal complex is not particularly limited as long as it is formed by reacting a radioactive metal with a ligand having a structure composed of those appropriately selected from the aforementioned first atomic group, a chelate site and a modified site. The radioactive metal complex is preferably formed by reacting a ligand having a structure represented by the following formula (ii) with a radioactive metal.

A-B-C ··· (ii)

In the formula (ii), A is a chelate site represented by the following formula (iia).

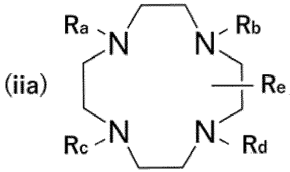

In the formula (iia), Ra, Rb and Rc are each independently a group consisting of -(CH₂)ₚCOOH, -(CH₂)ₚC₅H₅N, - (CH₂)ₚPO₃H₂, -(CH₂)ₚCONH₂ or -(CHCOOH)(CH₂)ₚCOOH, p is an integer of not less than 0 and not more than 3, one of Rd and Re is a binding site (*) with B, and other is a hydrogen atom or a group consisting of -(CH₂)ₚCOOH, - (CH₂)ₚC₅H₅N, -(CH₂)ₚPO₃H₂, - (CH₂)ₚCONH2 or, -(CHCOOH) (CH₂)ₚCOOH, and p is an integer of not less than 0 and not more than 3.

In the formula (ii), B is represented by the following formula (iib).

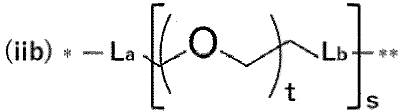

In the formula (iib), La and Lb are each independently a bond linker containing at least an amide bond or a thiourea bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

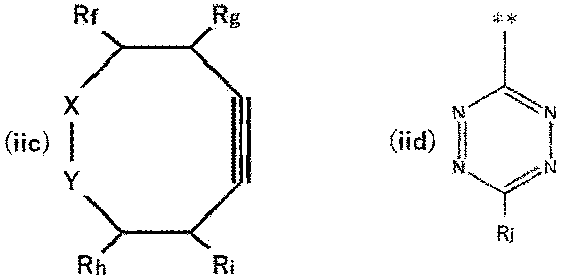

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbocycle, ** is a binding site with B, in the formula (iid), ** is a binding site with B, and Rj is a hydrogen atom, a methyl group, a phenyl group or a pyridyl group.

As the ligand for A, a DOTA derivative of the above-mentioned formula (iia) wherein Ra to Rd are -(CH₂)ₚCOOH, p is 1, Re is a binding site with B; or DO3A derivative or DOTAGA derivative wherein Ra to Rc are -(CH₂)ₚCOOH, p is 1, Rd is a binding site (*) with B, and Re is a hydrogen atom is more preferred.

In the formula (ii), a DOTA-PEGt-DBCO derivative wherein A is the above-mentioned DOTA derivative, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B; or a DOTA-PEGt-Tz derivative wherein, in B, La is a bond linker containing a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is a tetrazine derivative represented by the formula (iid), is further more preferred.

In the formula (ii), a DO3A-PEGt-DBCO derivative wherein A is the above-mentioned DO3A derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further more preferred.

In the formula (ii), a DOTAGA-PEGt-DBCO derivative wherein A is the above-mentioned DOTAGA derivative, in B, La is a bond linker containing an amide bond or a thiourea bond and having not less than 1 and not more than 50 carbon atoms, s is 0 or 1, when s is 1, t is an integer of not less than 0 and not more than 30, Lb is a bond linker containing an amide bond or a thiourea bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is further more preferred.

The matters applicable in common to each of the aforementioned embodiments are described below. In the peptide-modified antibody used in the present invention, a specific site of the antibody is specifically modified by the peptide, preferably the Fc region (constant region) of the antibody is specifically modified, and particularly preferably the lysine residue in the Fc region of the antibody is modified site-specifically. That is, in the present invention, a step of reacting a peptide with an antibody to obtain a peptide-modified antibody (antibody modification step) may be provided prior to the labeling step.

The antibody used in the present invention may be a polypeptide containing an Fc region, and may be a monoclonal antibody or a polyclonal antibody. A monoclonal antibody is preferred. Monoclonal antibody also includes artificially-modified genetically-modified antibodies such as antibody variants (chimeric antibody, humanized antibody, etc.).

The peptide used in the present invention may be a chain peptide or a cyclic peptide as long as it modifies the Fc region of the antibody in a site-specific manner, and a cyclic peptide is preferred. It preferably includes an amino acid sequence consisting of 13 to 17 amino acid residues represented by the following formula (i). In the following description of the amino acid sequence, the left side of the page of the amino acid sequence indicates the N-terminal side, and the right side of the page of the amino acid sequence indicates the C-terminal side. The binding position of the second atomic group linked to the peptide is not particularly limited as long as a click reaction can occur with the first atomic group. From the aspect of improving reactivity, the N-terminal or C-terminal of the peptide preferably has a second atomic group, and the N-terminal of the peptide more preferably has a second atomic group.

(Xa)-Xaa1-(Xb)-Xaa2-(Xc)-Xaa3-(Xd) ··· (i)

In the above-mentioned formula (i), Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently an amino acid residue derived from an amino acid having a thiol group in the side chain, and they are bonded via a disulfide bond or their sulfide groups are bonded via a linker, or
one is an amino acid residue derived from an amino acid having a thiol group in the side chain and the other is an amino acid residue derived from an amino acid having a haloacetyl group in the side chain, and they are bonded via a thioether bond, and Xaa2 is a lysine residue, arginine residue, cysteine residue, aspartic acid residue, glutamic acid residue, 2-aminosuberic acid, or diamino propionic acid, and preferably modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.

The numbers a, b, c and d in the formula (i) are not particularly limited as long as they are within the aforementioned range. From the aspect of binding stability between the peptide and the antibody, a+b+c+d≤14 being the condition wherein a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

Xaa1 and Xaa3 are amino acid residues derived from an amino acid having a thiol group in the side chain, and may be the same or different. Examples of the amino acid having a thiol group in the side chain include cysteine and homocysteine. Such amino acid residues are preferably bonded by a disulfide bond, or a sulfide group is preferably bonded thereto via a linker shown by the following formula (4). In the formula (4), the broken line indicates the binding part with the sulfide group.

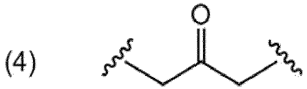

Instead of the aforementioned combination of Xaa1 and Xaa3, one of Xaa1 and Xaa3 may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

A specific amino acid sequence of the peptide represented by the formula (i) is, for example, the peptide described in WO 2016/186206, WO 2017/217347 or WO 2018/230257, and these can also be used.

Among these, the amino acid sequence of the peptide preferably has any one of the following sequences (1)-(14), more preferably the following sequence (1), (2), (13) or (14). The presence of such amino acid sequence can enhance the binding affinity between the peptide and the antibody (e.g., human IgG). In the following amino acid sequences (1)-(14), (Xaa2) is a lysine residue, a cysteine residue, an aspartic acid residue, a glutamic acid residue, a 2-aminosuberic acid, or a diamino propionic acid, and is preferably modified by a crosslinking agent. (Xaa1) and (Xaa3) both show homocysteine residues. In the following amino acid sequences (1)-(14), the amino acids other than (Xaa1), (Xaa2) and (Xaa3) are indicated by one-letter abbreviations.

(1) DCAYH(Xaa2)GELVWCT
(2) GPDCAYH(Xaa2)GELVWCTFH
(3) RCAYH(Xaa2)GELVWCS
(4) GPRCAYH(Xaa2)GELVWCSFH
(5) SPDCAYH(Xaa2)GELVWCTFH
(6) GDDCAYH(Xaa2)GELVWCTFH
(7) GPSCAYH(Xaa2)GELVWCTFH
(8) GPDCAYH(Xaa2)GELVWCSFH
(9) GPDCAYH(Xaa2)GELVWCTHH
(10) GPDCAYH(Xaa2)GELVWCTFY
(11) SPDCAYH(Xaa2)GELVWCTFY
(12) SDDCAYH(Xaa2)GELVWCTFY
(13) RGNCAYH(Xaa2)GQLVWCTYH
(14) G(Xaa1)DCAYH(Xaa2)GELVWCT(Xaa3)H

The peptide to be used in the present invention can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method or the like. In the synthesis of the peptide, where necessary, the functional groups of the amino acids to be used may be protected. These methods can be performed according to the method described in, for example, WO 2017/217347 and WO 2018/230257.

As a method for introducing the aforementioned second atomic group into peptide in the present invention, an introduction method including obtaining a peptide having a desired amino acid sequence by the aforementioned method, dissolving the peptide in a solution containing a solubilizing agent and a reducing agent and where necessary, an acid, adding an organic solvent solution of an atomic group containing an azide group or TCO as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding a peptide to an antibody to obtain a peptide-modified antibody can be performed using, for example, a crosslinking agent. A crosslinking agent is a chemical substance for linking a peptide and an antibody by a covalent bond. Examples thereof include a crosslinking agent preferably containing two or more succinimidyl groups such as disuccinimidyl glutarate (DSG), disuccinimidyl suberate (DSS) and the like, a crosslinking agent consisting of a compound containing two or more imidic acid moieties such as dimethyl adipimidate and the like, or a salt thereof, a crosslinking agent consisting of a compound having a disulfide bond such as dimethyl 3,3'-dithiobispropionimidate, dithiobissuccinimidylpropionic acid, and the like, or a salt thereof, and the like. Using such crosslinking agent, a crosslinking reaction can be caused between an amino acid residue of Xaa2 in the peptide and an antibody. As the site where the crosslinking reaction occurs in the antibody, when human IgG (e.g., trastuzumab) is used as the antibody, site-specific binding occurs via a crosslinked structure, between the amino acid residue of Xaa2 and at least one of the Lys246 residue and the Lys248 residue according to Eu numbering in trastuzumab. These Lys residues are present in the Fc region of human IgG, and even if the antibody is other than trastuzumab, those skilled in the art can align the amino acid sequence of the antibody and identify the corresponding Lys residue.

The method for binding the peptide to the antibody can be performed, for example, by dispersing the aforementioned peptide, an antibody, a crosslinking agent, and a catalyst as necessary in an appropriate buffer at not less than 10°C and not more than 30°C. The reaction time may be about not less than 10 min to about 2 hr. The molar ratio at the time of reaction of the peptide and the antibody is preferably within the range of 1:1-20:1 as peptide:antibody.

In the peptide-modified antibody obtained through the above steps, at least not less than one molecule of a peptide may be bonded per one molecule of an antibody (preferably antibody with IgG as the immunoglobulin class). To maintain the activity of the antibody itself (antigen recognition action, neutralizing action, complement activating action, opsonin action), a peptide is preferably bonded site-specifically to the Fc region (constant region) of the antibody. In the peptide-modified antibody of the present invention, one or two molecules of the peptide is/are preferably bonded per one molecule of the antibody.

The above-mentioned peptide-modified antibody obtained through the above steps is a mixture containing an antibody in which one molecule of peptide is bound to one molecule of antibody (hereinafter referred to as "monovalent antibody") and an antibody in which two molecules of peptide are bound to one molecule of antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is preferable to use a column filled with various fillers, and it is more preferable to use a column filled with a filler suitable for separation and purification of protein such as antibody and the like.

As the radioactive metal coordinated in an ionic state to the radioactive metal complex, a metal nuclide that emits α-ray, β-ray, γ-ray, or a combination thereof can be used. Examples of such radioactive metal nuclide include alkali metal, alkaline earth metal, lanthanoid, actinide, transition metal, and radioisotope of metals other than these metals. Of these, from the aspect of being commercially available and improving complex formation, ⁴⁴Sc, ⁵¹Cr, ⁵⁷Co, ⁵⁸Co, ⁶⁰Co, ⁵⁹Fe, ⁶⁷Ga, ⁶⁸Ga, ⁶⁴Cu, ⁶⁷Cu, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁷Th or ²²⁵Ac is preferably used as a radioactive metal nuclide. These radioactive metals can be produced according to a conventional method, and are preferably obtained as a solution containing the radioactive metal in an ionized state.

When the radioactive metal-labeled antibody is used for the treatment of a disease, α ray-emitting nuclide or β ray-emitting nuclide is preferably used as the radioactive metal in order to enhance the treatment effect. The α ray-emitting nuclide may be any nuclide that emits α ray in the decay process of a radioactive metal. Specifically, ²¹²Bi, ²¹³Bi, ²²⁷Th, ²²⁵Ac, or the like is preferably used. More preferred is ²²⁷Th or ²²⁵Ac, and further preferred is ²²⁵Ac. The β ray-emitting nuclide may be any nuclide that emits β ray in the decay process of a radioactive metal. In detail, ⁶⁰Co, ⁵⁹_{Fe}, 64_{Cu}, ⁶⁷Cu, ⁸⁹Sr, ⁹⁰Y, ^{99m}Tc, ¹⁰³Ru, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰³Hg, ²¹²Bi, ²¹³Bi, ²¹²Pb, and the like are preferably used, and ⁶⁴Cu, ⁶⁷Cu, ⁸⁹Sr, ⁹⁰Y or ¹⁷⁷Lu is more preferably used.

When the radioactive metal-labeled antibody is used for the diagnosis of disease or detection of lesion, β⁺ ray-emitting nuclide, electron capture decay nuclide, or γ ray-emitting nuclide is preferably used as the radioactive metal in order to enhance the diagnosis performance. The β⁺ ray-emitting nuclide may be any nuclide that emits positron in the decay process of a radioactive metal, and ⁴⁴Sc, ⁵⁸Co, ⁶⁸Ga, ⁶⁴Cu, ⁸⁹Zr, and the like are preferably used, and ⁶⁴Cu or ⁸⁹Zr is more preferred. The electron capture decay nuclide may be any nuclide that emits Auger electron or characteristic X-ray in the decay process of a radioactive metal, and ⁵¹Cr, ⁵⁷Co, ⁵⁸Co, ⁶⁷Ga, ⁶⁸Ga, ⁶⁴Cu, ⁸⁹Zr, ¹¹¹In, ¹⁸⁶Re, ²⁰¹Tl, ¹⁹⁷Hg and the like are preferably used. The γ ray-emitting nuclide may be any nuclide that emits γ ray by γ decay. As the nuclide that emits γ ray by γ decay, ^{99m}Tc, ⁶⁸Ga or ²⁰¹Tl is preferably used.

For example, when a radioactive metal coordinated in an ionic state to the radioactive metal complex is selected based on the ion radius, a radioactive metal with an ion radius of about 70-130 pm includes ⁶⁷Ga, ⁶⁸Ga, ⁶⁴Cu, ⁶⁷Cu, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁵Ac, and the like. These can preferably form a radioactive metal complex with a ligand having a chelate site with a structure represented by the above-mentioned formulas (A) to (K).

For example, when the radioactive metal-labeled antibody is used for the treatment of a disease and ²²⁵Ac, ¹⁷⁷Lu or ⁹⁰Y is used as a radioactive metal, it is preferable to use a ligand having a chelate site with a structure represented by any of the above-mentioned formulas (A) or (D) to (I), it is more preferable to use a ligand having a chelate site with a structure represented by the above-mentioned formula (A), (D) or (F), and it is further preferable to use a ligand having a chelate site with a structure represented by the above-mentioned formula (A). When the radioactive metal-labeled antibody is used for the diagnosis of disease or detection of lesion and ⁸⁹Zr or ¹¹¹In is used as a radioactive metal, it is preferable to use a ligand having a chelate site with a structure represented by any of the above-mentioned formulas (A), (C) and (K), and it is further preferable to use a ligand having a chelate site with a structure represented by the above-mentioned formula (A).

As the immunoglobulin class of the antibody used in the present invention, any class of IgG, IgA, IgM, IgD or IgE can be used without particular limitation. Among these, IgG is preferably used because it is the main class in the secondary response of the immune response, is most abundant in blood, and shows high recognition specificity for antigens. Furthermore, mammalian IgG is preferable, and specific examples of the mammal include primates such as human, chimpanzee and the like; experimental animals such as rat, mouse, rabbit and the like; domestic animals such as swine, bovine, horse, sheep, goat and the like; and pet animals such as dog and cat and the like. In addition, human IgG or rabbit IgG is further preferable, and human IgG is further more preferable. The subclass of these antibodies is not particularly limited. For example, when human IgG is used, it may be at least one of IgG1, IgG2, IgG3 and IgG4, and IgG1, IgG3 or IgG4 is preferred.

In the radioactive metal-labeled antibody obtained by the aforementioned production method, a specific site of an antibody is specifically modified with a peptide. A radioactive metal complex is directly or indirectly linked to the peptide, and preferably has a binding site-which is formed by a click reaction-between the peptide and the radioactive metal complex. The binding site is preferably a chemical structure derived from the first atomic group that the radioactive metal complex has and the second atomic group linked to the peptide. As such chemical structure, for example, the binding site may have a structure containing a substituted triazole skeleton or a structure containing a substituted pyridazine skeleton. As the structure containing the aforementioned substituted skeleton, for example, a structure in which a structure containing at least one of a substituent, an aliphatic ring and an aromatic ring is bonded to a triazole skeleton or a pyridazine skeleton, and which has a binding site with a modified site or a chelate site and a binding site with a peptide can be mentioned.

As the structure of a specific binding site, for example, when the first atomic group and the second atomic group are a combination of an atomic group containing DBCO and an atomic group containing an azide group, a structure containing a triazole skeleton shown by the following formula (10a) or formula (10b) is formed, depending on the reaction reagent used. Since these are isomers, they may be contained at any ratio. When the first atomic group and the second atomic group are a combination of an atomic group containing 1,2,4,5-tetrazine, and an atomic group containing TCO, a structure containing a pyridazine skeleton shown by the following formula (10c) can be formed, depending on the reaction reagent used. In the formula (10a) and the formula (10b), R_{1A} is a binding site with a modified site or a chelate site, R_{2A} is a binding site with a peptide. In the formula (10c), one of R_{3A} and R_{4A} is a hydrogen atom, a methyl group, a phenyl group or a pyridyl group, and the other is a binding site with a modified site or a chelate site, and R_{5A} is a binding site with a peptide.

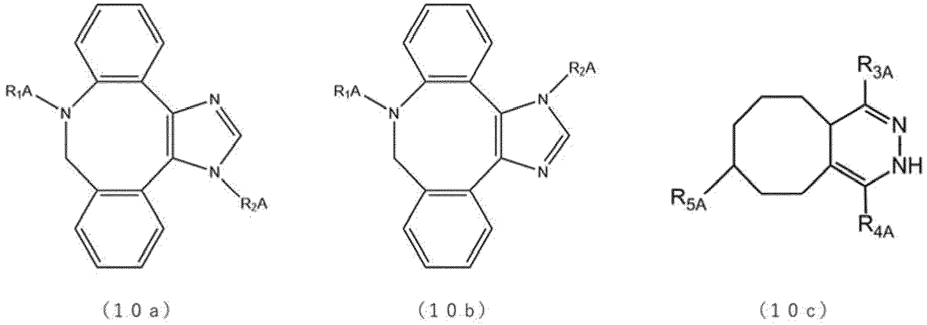

The radioactive metal-labeled antibody may also be used as it is or after purification for the preparation of a radioactive pharmaceutical composition containing the radioactive metal-labeled antibody as the active ingredient. The radioactive pharmaceutical composition refers to a composition containing a radioactive metal-labeled antibody or a derivative thereof, and in a form suitable for administration to a living body. The radioactive pharmaceutical composition can be produced, for example, by dissolving a radioactive metal-labeled antibody produced by the aforementioned method in a solvent mainly composed of water and substantially isotonic with a living body. In this case, the radioactive pharmaceutical composition is preferably in the form of an aqueous solution, and may contain other pharmaceutically acceptable components as necessary. A radioactive pharmaceutical composition is orally or parenterally, for example, intravenously, subcutaneously, intraperitoneally, intramuscularly, or the like, administered to a living body, and is used for treatment of a disease, diagnosis of a disease, detection of a lesion, or the like.

Examples of the substituent that can be used to substitute the above-mentioned formulas (A) to (J), the triazole skeleton-containing structure, and the pyridazine skeleton-containing structure include a halogen atom, a saturated or unsaturated alkyl group, a hydroxy group, an aldehyde group, a carboxy group, an acyl group, an amino group, a nitro group, an ester group, an isothiocyanate group, a thioxy group, a cyano group, an amide group, an imide group, a phosphate group, a phenyl group, a benzyl group, a pyridyl group, and the like. One of these substituents may be used alone for substitution, or two or more of these substituents may be used in combination.

### EXAMPLE

The present invention is described in more detail in the following by way of examples. However, the scope of the present invention is not limited by the examples. In the Tables below, the column with "-" indicates no performance.

### [Examples 1 to 4]

### (1-1. Complex formation step)

The structures of the ligands used in these Examples are shown in the following formulas (L1-1) to (L1-3). DO3A-DBCO represented by the formula (L1-1) was synthesized according to the method described in Liang Y, Jiang X, Yuan R, Zhou Y, Ji C, Yang L et al. Metabolism-Based Click-Mediated Platform for Specific Imaging and Quantification of Cell Surface Sialic Acids. Anal Chem. Jan 3; 89(1): 538-543. (2017). DOTA-DBCO represented by the formula (L1-2) was synthesized according to the method described in Wang H, Wang R, Cai K, He H, Liu Y, Yen J et al. Selective in vivo metabolic cell-labeling-mediated cancer targeting. Nat Chem Biol. Apr; 13(4): 415-424.(2017). As DO3A-PEG4-DBCO represented by the formula (L1-3), a commercially available product manufactured by Iris Biotech GmbH was used. These ligands were dispersed in 0.1 mol/L sodium acetate buffer (pH 6.0) as a solvent to give dispersions containing 1.7 mmol/L ligand. A reaction mixture of the dispersion (0.0025 mL), and ²²⁵Ac ion-containing solution (0.2 mol/L aqueous hydrochloric acid solution, radioactivity concentration 160 MBq/mL, prepared from one produced by Oak Ridge National Laboratory, liquid amount 0.0025 mL) 0.4 MBq (calculated by attenuation from the amount of radioactivity at test date and time) as a radioactive metal source was reacted under heating conditions to give a ²²⁵Ac complex solution. The molar ratio of the ligand and the radioactive metal ion was ligand:²²⁵Ac ion = 3000:1, and the heating temperature of the reaction mixture was set to 70°C, and the heating time was set to 90 min.

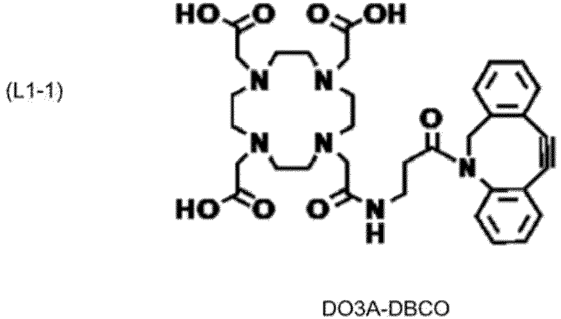

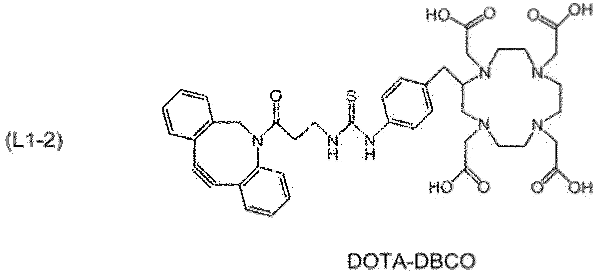

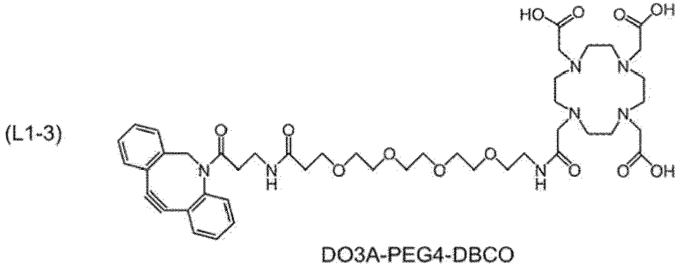

The radiochemical purity of the obtained ²²⁵Ac complex was measured by the following method. That is, a part of the ²²⁵Ac complex solution was developed by thin layer chromatography (manufactured by Agilent, model number: SGI0001, eluent: acetonitrile/water mixed solution (volume ratio 1:1)), and then measured by radio γ-TLC Analyzer (manufactured by raytest, MODEL GITA Star). The percentage of the radioactivity (count) of the peak detected near the origin with respect to the detected total radioactivity (count) was defined as the radiochemical purity (%) of the ²²⁵Ac complex. As a result, the radiochemical purity of the ²²⁵Ac complex was 89-99%. The obtained ²²⁵Ac complex solution was directly used for the labeling step.

### (1-2. Antibody modification step)

Separately, a peptide was produced by the method described in WO 2017/217347 to obtain a peptide containing 17 amino acid residues represented by the following formula (P3). The amino acid sequence of this peptide was the same as the sequence in which Xaa2 of SEQ ID NO: (2) was a lysine residue, and the side chain terminal amino group of the lysine residue was modified with the structure shown by R₁. In addition, two cysteine residues form a disulfide bond with each other, and to the N-terminal of the peptide is added ethyl azide as an atomic group containing an azide group, which is the second atomic group, via a linker structure having diglycolic acid and eight PEGs. 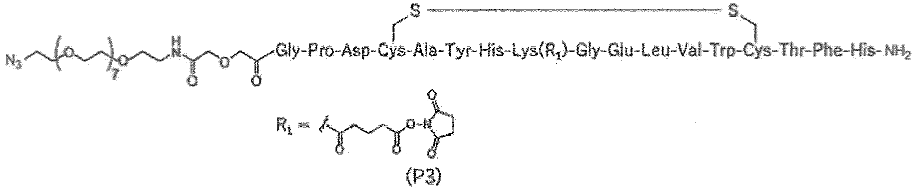 wherein in the formula (P3), Gly is glycine, Pro is proline, Asp is aspartic acid, Cys is cysteine, Ala is alanine, Tyr is tyrosine, His is histidine, Glu is glutamic acid, Leu is leucine, Val is valine, Trp is tryptophan, Phe is phenylalanine.

A mixture of the peptide and a human IgG antibody (rituximab; manufactured by Roche, or trastuzumab; manufactured by Roche) in a sodium acetate buffer (pH 6.0) was reacted at room temperature for 30 min to give a solution containing a peptide-modified antibody. The peptide-modified antibody has an Fc region of the antibody site-specifically modified by the above-mentioned peptide.

### (2. Peptide-modified antibody separation step)

The aforementioned peptide-modified antibody was diluted with 1 mol/L sodium acetate buffer (pH 6.0), added to Protein A column (manufactured by GE Healthcare, HiTrap MabSelect SuRe), and a 0.05 mol/L sodium acetate buffer (pH 5.7) containing 0.15 mol/L sodium chloride was flown. A solution containing a divalent antibody was recovered, and the concentration was adjusted such that the concentration of the divalent antibody contained in the recovered fraction was 15 mg/mL. Thereafter, 0.05 mol/L sodium acetate buffer (pH 3.5) containing 0.15 mol/L sodium chloride was flown, a solution containing a monovalent antibody was recovered, and the concentration was adjusted such that the concentration of the unmodified antibody and monovalent antibody contained in the recovered fraction was 17-40 mg/mL.

### (3. Labeling step)

A solution of the ²²⁵Ac complex obtained in each of the aforementioned steps, and a solution containing a peptide-modified antibody (monovalent antibody) were each added without purification to 0.02 mol/L ascorbic acid-containing 0.09 mol/L sodium acetate buffer, and a click reaction was performed at 37°C for 120 min to give ²²⁵Ac complex-labeled antibodies of Examples 1 to 4. The amount of the ²²⁵Ac complex and the amount of the peptide-modified antibody (monovalent antibody) were 43 µmol and 46 µmol (Example 1), respectively, or both 100 µmol (Examples 2 to 4), and the molar ratio of the first atomic group (DBCO) and the second atomic group (azide) was about 1:1. The reaction rate (%) of the unpurified ²²⁵Ac complex-labeled antibody of the Example is shown in the following Table 1. Here, the reaction rate (%) means the radiochemical purity (%) of the ²²⁵Ac complex-labeled antibody with respect to the labeling rate (%) in the complex formation step, and the labeling rate (%) means the amount of radioactivity (%) of the ²²⁵Ac complex with respect to the charged radioactivity amount.

Furthermore, a solution of the ²²⁵Ac complex-labeled antibody obtained by reacting at 37°C for 2 hr was purified using ultrafiltration filter (manufactured by Merck, model number: UFC505096). The radiochemical purity (RCP) and the radiochemical yield (RCY) of the ²²⁵Ac complex-labeled antibody after purification are shown in the following Table 1.

The measurement method of the radiochemical purity and radiochemical yield of the ²²⁵Ac complex-labeled antibody was as follows. That is, thin layer chromatography (manufactured by Agilent, model number: SGI0001, developing solvent was mixed solution of acetonitrile:0.1 mmol/L EDTA solution (volume ratio 1:1)) was measured by radio γ-TLC Analyzer (manufactured by raytest, MODEL GITA Star), and the percentage of the radioactivity (count) of the peak detected near the origin to the total radioactivity (count) detected was defined as the radiochemical purity (%). In addition, the percentage of the radioactivity (radioactivity level calculated from the count measured by γ ray spectrometer (Ge semiconductor detector: GMX10P4-70 (manufactured by ORTEC), Multi Channel Analyzer: M7-000 (manufactured by SEIKO EG&G), data processing: Spectrum Navigator:DS-P300 (manufactured by SEIKO EG&G) and Gamma Studio:DS-P600 (manufactured by SEIKO EG&G)) recovered after ultrafiltration purification with respect to the total radioactivity (similar to the above, the radioactivity level calculated from the count measured by γ ray spectrometer) added at the start of the labeling step was defined as the radiochemical yield (%).

### [Example 5]

### (1-1. Complex formation step)

In this Example, a ²²⁵Ac complex solution was obtained in the same manner as in Example 1 except that a ligand with the structure shown by the following formula (L2) was used. DOTA-PEG7-Tz represented by the formula (L2) was synthesized according to the method described in Poty S, Membreno R, Glaser JM, Ragupathi A, Scholz WW, Zeglis BM et al. The inverse electron-demand Diels-Alder reaction as a new methodology for the synthesis of 225Ac-labelled radioimmunoconjugates. Chem Commun (Camb). Mar 8; 54(21):2599-2602. (2018).

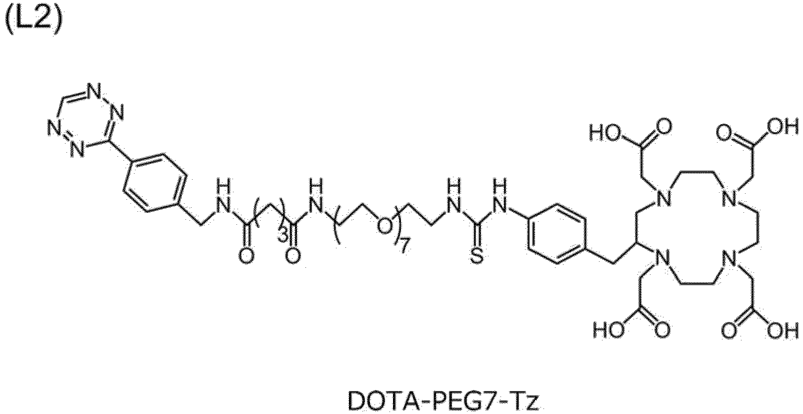

### (1-2. Antibody modification step)

The peptide used in this Example was a peptide containing 17 amino acid residues represented by the following formula (P4). The amino acid sequence of this peptide was the same as the sequence in which Xaa2 of SEQ ID NO: (2) was a lysine residue, and the side chain terminal amino group of the lysine residue was modified with the structure shown by R2. In addition, the thiol groups of the two cysteine residues are linked to each other by the linker represented by the above-mentioned formula (4), and the N-terminal of the peptide had a linker structure including 5 PEGs, a cysteine residue substituted by a structure shown by R₁, in which residue a thiol group of the side chain contains the second atomic group TCO, two glutamic acid residues, and an acetyl group in this order when viewed from the N-terminal side. 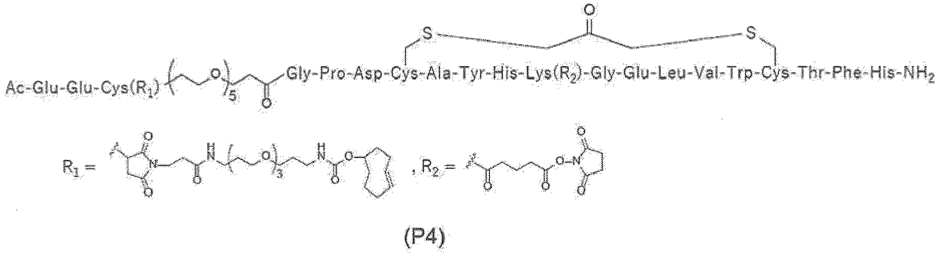 wherein in the formula (P4), Gly is glycine, Pro is proline, Asp is aspartic acid, Cys is cysteine, Ala is alanine, Tyr is tyrosine, His is histidine, Glu is glutamic acid, Leu is leucine, Val is valine, Trp is tryptophan, Phe is phenylalanine.

A mixture of the peptide and a human IgG antibody (trastuzumab; manufactured by Roche) in a sodium acetate buffer (pH 6) was reacted at room temperature for 30 min to give a solution containing a peptide-modified antibody.

### (2. Labeling step)

A click reaction was performed in the same manner as in Example 1 to give an ²²⁵Ac complex-labeled antibody. The amount of the ²²⁵Ac complex and the amount of the peptide-modified antibody were both 100 µmol, and the molar ratio of the first atomic group (1,2,4,5-tetrazine) and the second atomic group (TCO) was 1:1. The reaction rate (%) of the unpurified ²²⁵Ac complex-labeled antibody of the Example is shown in the following Table 1. Here, the reaction rate (%) means the radiochemical purity (%) of the ²²⁵Ac complex-labeled antibody with respect to the labeling rate (%) in the complex formation step, and the labeling rate (%) means the amount of radioactivity (%) of the ²²⁵Ac complex with respect to the charged radioactivity amount. In addition, the radiochemical purity (RCP) and the radiochemical yield (RCY) of the ²²⁵Ac complex-labeled antibody after purification using an ultrafiltration filter in the same manner as in Example 1 are shown in the following Table 1.

**[Table 1]**

| | ligand (A) having first atomic group | antibody | second atomic group (B) | molar ratio (A) : (B) | reaction rate | | | | after purification | |
|---|---|---|---|---|---|---|---|---|---|---|
| | | | | | room temperature reacted for 10 min (%) | 37°C reacted for 15 min (%) | room temperature reacted for 1 hr (%) | 37°C reacted for 2 hr (%) | RCP (%) | RCY (%) |
| Ex. 1 | DO3A-PEG4-DBCO | rituximab | azide | 1:1 | - | - | - | 49 | 94 | 36 |
| Ex. 2 | DO3A-PEG4-DBCO | trastuzumab | azide | 1:1 | - | 54 | - | 62 | 99 | 54 |
| Ex. 3 | DO3A-DBCO | trastuzumab | azide | 1:1 | - | 14 | - | 19 | 93 | 12 |
| Ex. 4 | DOTA-DBCO | trastuzumab | azide | 1:1 | - | 53 | - | 54 | 99 | 54 |
| Ex. 5 | DOTA-PEG7-Tz | trastuzumab | TCO | 1:1 | 65 | - | 65 | - | 98 | 45 |

As shown in Table 1, it is clear that, in the Examples in which the complex and the antibody were reacted after the complex formation step, the labeling reaction proceeded under mild reaction conditions without excessive heating of the antibody, and the radioactive metal is superior in the antibody labeling efficiency.

### [Examples 6 and 7] Production of Tmab, Rmab using ²²⁵Ac-labeled DOTAGA-DBCO

### (1. Complex formation step)

The structure of the ligand used in this Example is represented by the following formula (L1-4). DOTAGA-DBCO represented by the formula (L1-4) was produced based on the method described in Bernhard et al. DOTAGA-Anhydride: A Valuable Building Block for the Preparation of DOTA-Like Chelating Agents Chem. Eur. J. 2012, 18, 7834-7841. These ligands were dispersed in 0.1 mol/L sodium acetate buffer (pH 6.0) as a solvent to give a dispersion containing 1.7 mmol/L ligand. A reaction mixture of the dispersion (0.005 mL), and ²²⁵Ac ion-containing solution (0.2 mol/L aqueous hydrochloric acid solution, radioactivity concentration 320-340 MBq/mL, prepared from one produced by Oak Ridge National Laboratory, liquid amount: 0.005 mL) 1.6-1.7 MBq (calculated by attenuation from the radioactivity level at test date and time) as a radioactive metal source was reacted under heating conditions to give a ²²⁵Ac complex solution. The molar ratio of the ligand and the radioactive metal ion was ligand:²²³Ac ion = about 2500:1, and the heating temperature of the reaction mixture was set to 70°C, and the heating time was set to 30 min.

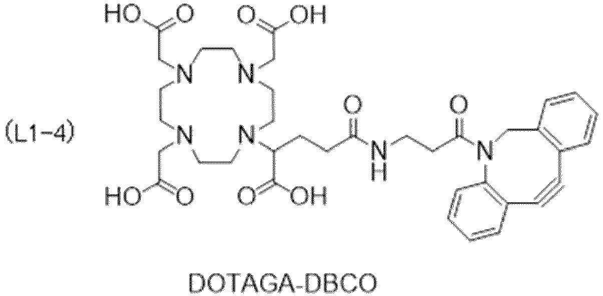

The radiochemical purity of the obtained ²²⁵Ac complex was measured in the same manner as in Example 1. As a result, the radiochemical purity was 90%. The obtained ²²⁵Ac complex solution was directly used for the next labeling step.

### (2. Labeling step)

A solution of the unpurified ²²⁵Ac complex obtained in the aforementioned step (1), and a solution containing a peptide-modified antibody (monovalent antibody) obtained in the same manner as in Example 1 were each added to 0.1 mol/L arginine-containing 0.1 mol/L histidine buffer (pH 6.0), and a click reaction was performed at 37°C for 120 min to give ²²⁵Ac complex-labeled antibody. The amount of the ²²⁵Ac complex and the amount of the peptide-modified antibody (monovalent antibody) were 85 nmol and 100 nmol (Examples 6 and 7), respectively, and the molar ratio of the first atomic group (DBCO) and the second atomic group (azide) was about 1:1.2. The reaction rate (%) of the unpurified ²²⁵Ac complex-labeled antibody is shown in the following Table 2. Here, the reaction rate (%) means the radiochemical purity (%) of the ²²⁵Ac complex-labeled antibody with respect to the labeling rate (%) in the complex formation step, and the labeling rate (%) means the amount of radioactivity (%) of the ²²⁵Ac complex with respect to the charged radioactivity amount.

Furthermore, a solution of the ²²⁵Ac complex-labeled antibody obtained by reacting at 37°C for 2 hr was purified using ultrafiltration filter (manufactured by Merck, model number: UFC505096). The radiochemical purity (RCP) and radiochemical yield (RCY) of the ²²⁵Ac complex-labeled antibody after purification are shown in the following Table 2.

The measurement method of the radiochemical purity and radiochemical yield of the ²²⁵Ac-labeled monovalent antibody was similar to that in Example 1.

**[Table 2]**

| | ligand (A) having first atomic group | antibody | second atomic group (B) | molar ratio (A) : (B) | reaction rate | after purification | |
|---|---|---|---|---|---|---|---|
| | | | | | 37°C reacted for 2 hr (%) | RCP (%) | RCY (%) |
| Ex. 6 | DOTAGA-DBCO | trastuzumab | azide | 1:1.2 | 77% | 93% | 71% |
| Ex. 7 | DOTAGA-DBCO | rituximab | azide | 1:1.2 | 77% | 95% | 73% |

### [Example 8] Production of Tmab using ⁸⁹Zr-labeled DOTAGA-DBCO (1. Complex formation step)

The structure of the ligand used in this Example is the same as that in the aforementioned formula (L1-4). This ligand was dispersed in DMSO as a solvent to give a dispersion containing 0.33 mmol/L ligand. A reaction mixture of the dispersion (0.030 mL), and ⁸⁹Zr ion-containing solution (0.1 mol/L aqueous hydrochloric acid solution, radioactivity concentration 181 MBq/mL, prepared from one manufactured by Nihon Medi-Physics Co., Ltd., liquid amount 0.33 mL) 60 MBq as a radioactive metal source was reacted under heating conditions to give a ⁸⁹Zr complex solution. The molar ratio of the ligand and the radioactive metal ion was ligand:⁸⁹Zr ion = about 250:1, and the heating temperature of the reaction mixture was set to 70°C, and the heating time was set to 60 min.

The radiochemical purity of the obtained ⁸⁹Zr complex was measured by the following method. That is, a part of the ⁸⁹Zr complex solution was developed by thin layer chromatography (manufactured by Agilent, model number: SGI0001, developing solvent: acetonitrile/water mixed solution (volume ratio 1:1)), and then measured by radio γ-TLC Analyzer (manufactured by raytest, MODEL GITA Star PS). The percentage of the radioactivity (count) of the peak detected near the origin with respect to the detected total radioactivity (count) was defined as the radiochemical purity (%) of the ⁸⁹Zr complex. As a result, the radiochemical purity of the ⁸⁹Zr complex was 90%. The obtained ⁸⁹Zr complex solution was used as it was in the labeling step.

### (2. Labeling step)

A solution of the unpurified ⁸⁹Zr complex obtained in the aforementioned step (1), and a solution containing a peptide-modified antibody (monovalent antibody) obtained in the same manner as in Example 1 were each added without purification to 0.1 mol/L arginine-containing 0.1 mol/L histidine buffer (pH 6.0), and a click reaction was performed at 37°C for 120 min to give ⁸⁹Zr complex-labeled antibody of Example 8. The amount of the ⁸⁹Zr complex and the amount of the peptide-modified antibody (monovalent antibody) were each 100 nmol, and the molar ratio of the first atomic group (DBCO) and the second atomic group (azide) was about 1:1. The reaction rate (%) of the unpurified ⁸⁹Zr complex-labeled antibody of the Example is shown in the following Table 3. Here, the reaction rate (%) means the radiochemical purity (%) of the ⁸⁹Zr complex-labeled antibody with respect to the labeling rate (%) in the complex formation step, and the labeling rate (%) means the amount of radioactivity (%) of the ⁸⁹Zr complex with respect to the charged radioactivity amount.

Furthermore, a solution of the ⁸⁹Zr complex-labeled antibody obtained by reacting at 37°C for 2 hr was purified using ultrafiltration filter (manufactured by Merck, model number: UFC505096). The radiochemical purity (RCP) and radiochemical yield (RCY) of the ⁸⁹Zr complex-labeled antibody after purification are shown in the following Table 3.

The measurement method of the radiochemical purity and radiochemical yield of the ⁸⁹Zr complex-labeled antibody was similar to that in Example 8.

**[Table 3]**

| | ligand (A) having first atomic group | antibody | second atomic group (B) | molar ratio (A) : (B) | reaction rate | after purification | |
|---|---|---|---|---|---|---|---|
| | | | | | 37°C reacted for 2 hr (%) | RCP (%) | RCY (%) |
| Ex. 8 | DOTAGA-DBCO | trastuzumab | azide | 1:1 | 52% | 93% | 43% |

### [Example 9] Production of Tmab using ⁸⁹Zr-labeled DOTA-DBCO (1-1. Complex formation step)

The structure of the ligand used in this Example is the same as that in the aforementioned formula (L1-2). This ligand was dispersed in DMSO as a solvent to give a dispersion containing 1 mmol/L ligand. A reaction mixture of the dispersion (0.1 mL), and ⁸⁹Zr ion-containing solution (0.1 mol/L aqueous hydrochloric acid solution, radioactivity concentration 450 MBq/mL, prepared from one manufactured by Okayama University, liquid amount 0.1 mL) 45 MBq as a radioactive metal source was reacted under heating conditions to give a ⁸⁹Zr complex solution. The molar ratio of the ligand and the radioactive metal ion was ligand:⁸⁹Zr ion = about 10000:1, and the heating temperature of the reaction mixture was set to 70°C, and the heating time was set to 60 min.

The radiochemical purity of the obtained ⁸⁹Zr complex was measured according to Example 1. As a result, the radiochemical purity of the ⁸⁹Zr complex was 99%.

### (1-2. Unreacted substance removal step)

The obtained ⁸⁹Zr complex solution was collected by high performance liquid chromatography (HPLC), and unreacted DOTA-DBCO was removed. The conditions of HPLC were as follows. detector: ultraviolet absorption spectrophotometer (measurement wavelength: 254 nm)/scintillation detector, column: XBridge C18 3.5 µm, 4.6×100 nm; manufactured by Waters, mobile phase A: 10 mmol/L histidine buffer pH 6.5, mobile phase B: acetonitrile for liquid chromatography, feed of mobile phase: the mixing ratio of the mobile phase A and mobile phase B was changed as follows to control the concentration gradient (A:B=90:10(0 min) →50:50(40 min) (vol%/vol%)), flow: 0.5 mL/min, collection peak retention time: about 32 min. The solvent was evaporated from the obtained collected solution to about 20 µL of the solution and the solution was used in the labeling step. The radiochemical yield (HPLC recovery rate) in the step of removing the unreacted substance of the ⁸⁹Zr complex-labeled antibody is shown in the following Table 4. In the method for measuring the radiochemical yield (HPLC recovery rate), the percentage of radioactivity in the collected solution was defined as the HPLC recovery rate (%) in the unreacted substance removal step with respect to the amount of radioactivity charged at the start of this step.

### (2. Labeling step)

A solution of the ⁸⁹Zr complex obtained in the aforementioned each step, and a solution containing a peptide-modified antibody (monovalent antibody) obtained in the same manner as in Example 1 were each added to 0.1 mol/L arginine-containing 0.1 mol/L histidine buffer (pH 6.0), and a click reaction was performed at 37°C for 120 min to give ⁸⁹Zr complex-labeled antibody. The labeling step reaction rate (%) of the unpurified ⁸⁹Zr complex-labeled antibody of the Example is shown in the following Table 4. Here, the labeling step reaction rate (%) means the amount of radioactivity (%) of the ⁸⁹Zr complex with respect to that of the HPLC collected solution.

Furthermore, a solution of the ⁸⁹Zr complex-labeled antibody obtained by reacting at 37°C for 2 hr was purified using ultrafiltration filter (manufactured by Merck, model number: UFC505096). The radiochemical purity (RCP) and radiochemical yield (RCY) of the ⁸⁹Zr complex-labeled antibody after purification are shown in the following Table 4.

The measurement method of the radiochemical purity and radiochemical yield of the ⁸⁹Zr complex-labeled antibody followed that in Example 1.

**[Table 4]**

| | ligand (A) having first atomic group | HPLC recovery rate | antibody | second atomic group (B) | labeling step reaction rate | after purification | |
|---|---|---|---|---|---|---|---|
| | | | | | 37°C reacted for 2 hr (%) | RCP (%) | RCY (%) |
| Ex. 9 | DOTA-DBCO | 57% | trastuzumab | azide | 73% | 98% | 39% |

While the present invention has been described above with reference to the embodiments, the present invention is not limited to the above-mentioned embodiments. Various changes that can be understood by those skilled in the art can be made to the constitution and details of the present invention within the scope of the present invention.

The contents disclosed in any publication cited herein, including patents and patent applications, are hereby incorporated in their entireties by reference, to the extent that they have been disclosed herein.

### INDUSTRIAL APPLICABILITY

According to the present invention, high labeling efficiency of radioactive metals for antibodies can be achieved even under mild reaction conditions.

This application is based on a patent application No. 2019-191561 filed in Japan (filing date: October 18, 2019), the contents of which are incorporated in full herein.

## Claims

1. A method for producing a radioactive metal-labeled antibody, comprising a step of conducting a click reaction of a radioactive metal complex and a peptide-modified antibody which is site-specifically modified with a peptide to produce the radioactive metal-labeled antibody, wherein
the click reaction is performed between a first atomic group of the radioactive metal complex and a second atomic group directly or indirectly linked to the peptide, and
the second atomic group is an atomic group containing an azide group, or an atomic group containing trans-cyclooctene.

2. The method for producing a radioactive metal-labeled antibody according to claim 1, wherein the peptide comprises an amino acid sequence represented by the following formula (i) and consisting of 13 to 17 amino acid residues, and has the second atomic group at the N-terminal or C-terminal of the peptide:
(Xa)-Xaa1-(Xb)-Xaa2-(Xc)-Xaa3-(Xd)... (i)
wherein Xa, Xb, Xc, and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently an amino acid residue derived from an amino acid having a thiol group in the side chain, and they are bonded via a disulfide bond or their sulfide groups are bonded via a linker, or
one is an amino acid residue derived from an amino acid having a thiol group in the side chain and the other is an amino acid residue derived from an amino acid having a haloacetyl group in the side chain, and they are bonded via a thioether bond, and Xaa2 is a lysine residue, an arginine residue, a cysteine residue, an aspartic acid residue, a glutamic acid residue, a 2-aminosuberic acid, or a diamino propionic acid.

3. The method for producing a radioactive metal-labeled antibody according to claim 1 or 2, further comprising a step of reacting the ligand with the radioactive metal to form the radioactive metal complex.

4. The method for producing a radioactive metal-labeled antibody according to claim 3, wherein the ligand and the radioactive metal are reacted by heating.

5. The method for producing a radioactive metal-labeled antibody according to claim 3 or 4, wherein the ligand comprises a chelate site coordinated to the radioactive metal, and a modified site to which the first atomic group is bonded.

6. The production method according to claim 5, wherein the chelate site is represented by any one of the following formulas (A) to (K): 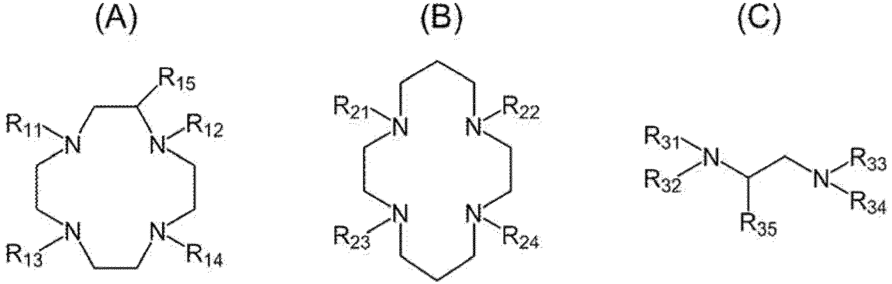 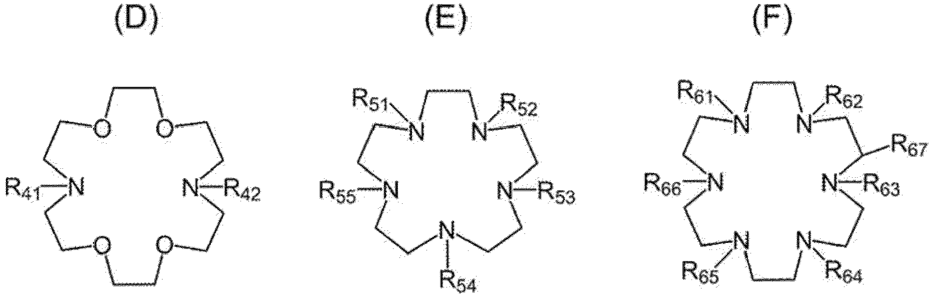 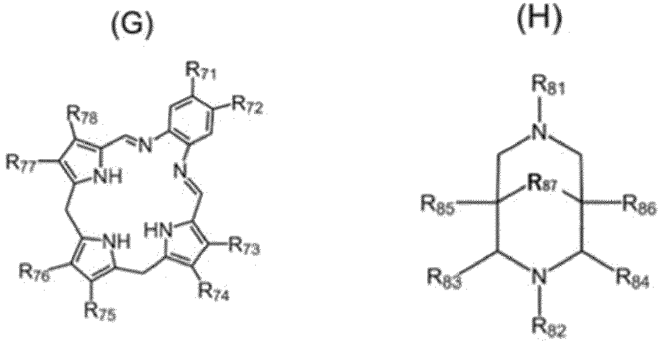 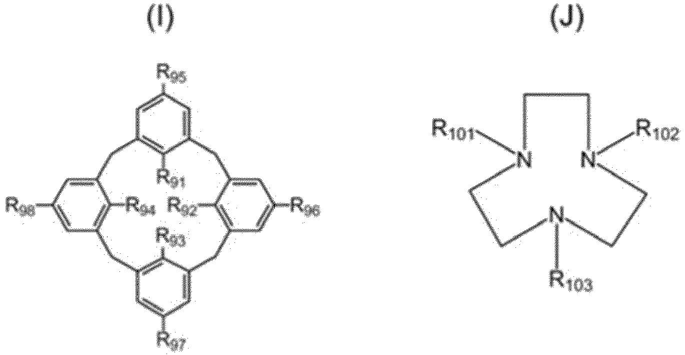 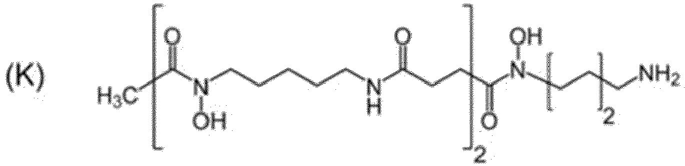 in the formula (A), R₁₁, R₁₃ and R₁₄ are each independently a group consisting of -(CH₂)ₚCOOH, -(CH₂) ₚC₅H₅N, -(CH₂) ₚPO₃H₂, - (CH₂)ₚCONH₂ or - (CHCOOH) (CH₂)ₚCOOH, one of R₁₂ and R₁₅ is a hydrogen atom, a carboxyl group, or a carboxyalkyl group having 2 or 3 carbon atoms, the other is a group bonded to the modified site, and p is an integer of not less than 0 and not more than 3,
in the formula (B), R₂₁, R₂₂, R₂₃ and R₂₄ are each independently a carboxyl group or a carboxyalkyl group having a carbon number of 2 or 3, and any one group of R₂₁, R₂₂, R₂₃ or R₂₄ is bonded to the modified site,
in the formula (C), R₃₁, R₃₂, R₃₃ and R₃₄ are each independently a group composed of an atomic group having a hydrogen atom and not less than 2 and not more than 10 carbon atoms, and optionally containing a nitrogen atom or an oxygen atom, and R₃₅ is a group bonded to the modified site,
in the formula (D), one of R₄₁ and R₄₂ is a group composed of an atomic group having a hydrogen atom and not less than 5 and not more than 20 carbon atoms and containing one or more kinds selected from nitrogen atom, oxygen atom and sulfur atom, and the other is a group bonded to the modified site,
in the formula (E), R₅₁, R₅₂, R₅₃, R₅₄ and R₅₅ are each independently a carboxyl group, or a carboxyalkyl group having a carbon atom number of 2 or 3, and any one group of R₅₁, R₅₂, R₅₃, R₅₄ or R₅₅ is bonded to the modified site,
in the formula (F), R₆₁, R₆₂, R₆₃, R₆₄, R₆₅ and R₆₆ are each independently a carboxyl group, or carboxyalkyl group having a carbon atom number of 2 or 3, and R₆₇ is a group bonded to the modified site,
in the formula (G), R₇₁ and R₇₂ are -O(CH₂CH₂O)ₙCH₃ (n is an integer of not less than 1 and not more than 5), R₇₃, R₇₅, R₇₆ and R₇₈ are each independently an alkyl group having a carbon atom number of not less than 1 and not more than 5, one of R₇₄ and R₇₇ is a hydroxyalkyl group having a carbon number of not less than 1 and not more than 5, and the other is a group bonded to the modified site,
in the formula (H), R₈₁ and R₈₂ are each independently an alkyl group having a carbon number of not less than 1 and not more than 5, a terminal of the alkyl group may be substituted by a pyridyl group substituted by one or more carboxyl groups, R₈₇ is -CHOH or -C=O, any one group of R₈₁, R₈₂ or R₈₇ is a group bonded to the modified site, R₈₃ and R₈₄ are optionally substituted pyridinyl groups, R₈₅ and R₈₆ are each independently -COORa, and Ra is an alkyl group having a carbon number of not less than 1 and not more than 5,
in the formula (I), R₉₁, R₉₂, R₉₃ and R₉₄ are each independently -OCH₂COOH, any one group of R₉₁, R₉₂, R₉₃ or R₉₄ is a group bonded to the modified site, and R₉₅, R₉₆, R₉₇ and R₉₈ are each independently an alkyl group having a carbon number of not less than 1 and not more than 6, and
in the formula (J), R₁₀₁, R₁₀₂ and R₁₀₃ are each independently a carboxyl group or a carboxyalkyl group having a carbon atom number of 2 or 3, or at least one of R₁₀₁, R₁₀₂ and R₁₀₃ in the formula (J) is a group bonded to the modified site and the other group is a carboxyl group, or a carboxyalkyl group having a carbon atom number of 2 or 3.

7. The method for producing a radioactive metal-labeled antibody according to any one of claims 1 to 6, wherein the radioactive metal is ⁴⁴Ss, ⁵¹Cr, ⁵⁷Co, ⁵⁸Co, ⁶⁰Co, ⁵⁹Fe, ⁶⁷Ga, ⁶⁸Ga, ⁶⁴Cu, ⁶⁷Cu, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁷Th or ²²⁵Ac.

8. The method for producing a radioactive metal-labeled antibody according to claim 7, wherein the radioactive metal is an α ray-emitting nuclide.

9. The method for producing a radioactive metal-labeled antibody according to any one of claims 1 to 8, wherein the click reaction is performed at 50°C or below.

10. The method for producing a radioactive metal-labeled antibody according to any one of claims 1 to 9, wherein the peptide-modified antibody is site-specifically modified with 1 molecule or 2 molecules of peptide per 1 molecule of the antibody.

11. A radioactive metal-labeled antibody site-specifically modified with a peptide, wherein
the radioactive metal complex is directly or indirectly linked to the peptide, and a triazole skeleton-containing structure represented by the following formula (10a) is present between the peptide and the radioactive metal complex, or a pyridazine skeleton-containing structure is present between the peptide and the radioactive metal complex: 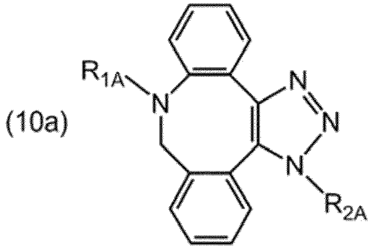 wherein R_{1A} is a binding site with a modified site or a chelate site, and R_{2A} is a binding site with a peptide.

12. The radioactive metal-labeled antibody according to claim 11, comprising a pyridazine skeleton-containing structure represented by the following formula (10c) between the peptide and the radioactive metal complex: 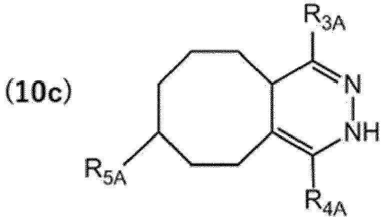 wherein one of R_{3A} and R_{4A} is a hydrogen atom, a methyl group, a phenyl group or a pyridyl group, and the other is a binding site with a modified site or a chelate site, and R_{5A} is a linking site with a peptide.

13. A chelate linker comprising a chelate site coordinated to a radioactive metal, and a modified site to which a first atomic group capable of click reaction is bonded, wherein the first atomic group is an atomic group represented by the following formula (1a) or (1b): 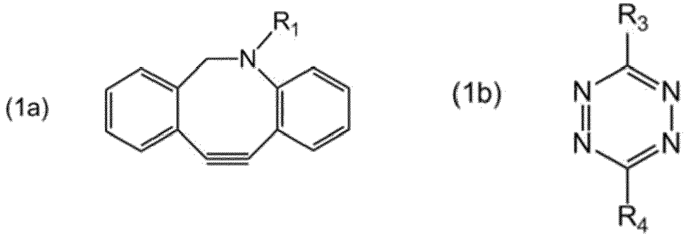 in the formula (1a), R₁ is a binding site with a modified site or a chelate site, and in the formula (1b), one of R₃ and R₄ is a binding site with a modified site or a chelate site, and the other is a hydrogen atom, a methyl group, a phenyl group or a pyridyl group.

14. A peptide-modified antibody represented by the following formula (i) and site-specifically modified by a peptide comprising an amino acid sequence consisting of 13 to 17 amino acid residues, which antibody comprising
a second atomic group capable of click reaction is linked to the N-terminal or C-terminal of the peptide, wherein the second atomic group is an atomic group represented by the following formula (2a) or (2b):
(Xa)-Xaa1-(Xb)-Xaa2-(Xc)-Xaa3-(Xd)... (i)
wherein Xa, Xb, Xc, and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently an amino acid residue derived from an amino acid having a thiol group in the side chain, and they are bonded via a disulfide bond or their sulfide groups are bonded via a linker, or
one is an amino acid residue derived from an amino acid having a thiol group in the side chain and the other is an amino acid residue derived from an amino acid having a haloacetyl group in the side chain, and they are bonded via a thioether bond, and Xaa2 is a lysine residue, an arginine residue, a cysteine residue, an aspartic acid residue, a glutamic acid residue, a 2-aminosuberic acid, or a diamino propionic acid, 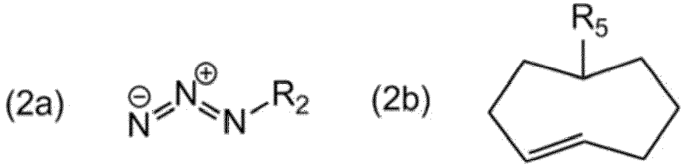
in the formula (2a), R₂ is a linking site with the N-terminal or C-terminal of the peptide, and in the formula (2b), R₅ is a linking site with the N-terminal or C-terminal of the peptide.

15. The antibody according to claim 14, wherein the peptide-modified antibody is site-specifically modified with 1 molecule or 2 molecules of the peptide per 1 molecule of the antibody.
